(19) Europäisches Patentamt

European Patent Office

Office européen des brevets

(11) **EP 1 471 444 A1**

(12) **EUROPEAN PATENT APPLICATION**
published in accordance with Art. 158(3) EPC

(43) Date of publication:
**27.10.2004 Bulletin 2004/44**

(51) Int Cl.7: **G06F 17/30**, C12Q 1/68,
C12N 15/00, G01N 33/50

(21) Application number: **03701003.0**

(22) Date of filing: **06.01.2003**

(86) International application number:
**PCT/JP2003/000011**

(87) International publication number:
**WO 2003/058500 (17.07.2003 Gazette 2003/29)**

(84) Designated Contracting States:
**AT BE BG CH CY CZ DE DK EE ES FI FR GB GR
IE IT LI LU MC NL PT SE SI SK TR**

(30) Priority: **28.12.2001 JP 2001402081**

(71) Applicant: **Celestar Lexico-Sciences, Inc.
Chiba-shi, Chiba 261-8501 (JP)**

(72) Inventors:
• **UEMURA, Yasuo, Celestar Lexico-Sciences, Inc.
Chiba-shi, Chiba 261-8501 (JP)**
• **HORAI, Hisayuki, Celestar Lexico-Sciences, Inc.
Chiba-shi, Chiba 261-8501 (JP)**

(74) Representative: **HOFFMANN EITLE
Patent- und Rechtsanwälte
Arabellastrasse 4
81925 München (DE)**

(54) **RNA SEQUENCE ANALYZER, AND RNA SEQUENCE ANALYSIS METHOD, PROGRAM AND RECORDING MEDIUM**

(57) A system according to the present invention is constituted so that an RNA sequence analyzer (100) that is an RNA sequence analyzer for analyzing sequence information, and an external system (200) that provides external databases related to the sequence information and the like, external analysis programs for homology searches, and the like are communicably connected to each other through a network (300).

EP 1 471 444 A1

## FIG.7

**Description**

TECHNICAL FIELD

The present invention relates to an RNA sequence analyzer, an RNA sequence analysis method, a program, and a recording medium. More specifically, the present invention relates to an RNA sequence analyzer, an RNA sequence analysis method, a program, and a recording medium for predicting RNA secondary structures from RNA sequences, and for predicting gene portions that is transcribed from DNA sequences.

BACKGROUND ART

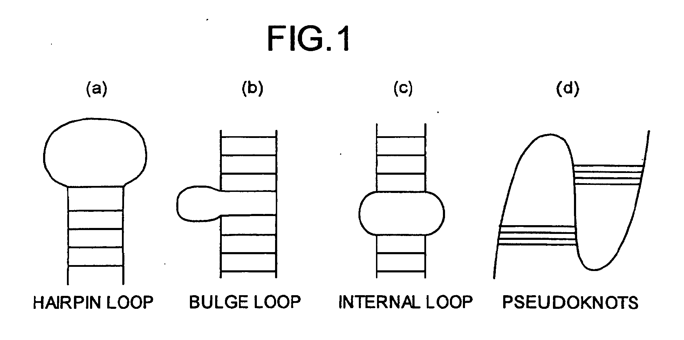

An RNA sequence consists of four type of bases of A (adenine), C (cytosine), G (guanine), and U (uracil). RNA sequences may have inverted repeat sequences in which complementary bases (A and U, G and C, and rarely G and U) are bound together to constitute a secondary structure. Fig. 1 illustrates a variety of structural subunits in topological forms that may join together to constitute complete pictures of RNA secondary structures. A continuous stretch of stacked base pairs is referred to as "stem", and a single strand sequence in-between some base-pairs is referred to as "loop". The loop on one end of the stem is referred to as "hairpin loop" (Fig. 1(a)). The loop inside the stem are referred to as "bulge loop" if the loop exists only on one side of the stem (Fig. 1(b)), and referred to as "internal loop" if the loops exist on both sides of the stem (Fig. 1(c)). If three or more stems are helically present, loops connecting those stems are referred to as "multi-branched loops". Fig. 1 also shows "pseudoknots" in which two stem bops cross each other (Fig. 1(d)). There has been several attempts to predict RNA secondary structures from RNA sequences using parsers for formal grammars (generative grammars). Although a method for predicting the secondary structure of the RNA sequence by subjecting the RNA sequence to parsing based on a formal grammar (generative grammar), palindromes cannot be represented in regular grammars. Normally, therefore, for the RNA secondary structure analysis, there is proposed a method for obtaining structure modeling (a structural topology representation) by subjecting the RNA sequence to parsing using tree adjoining grammars, context-free grammars (hereinafter "CFGs"), or the like.

For example, Yasuo Uemura et al.: "Tree adjoining grammars for RNA structure prediction" in *Theoretical Computer Science 210*, 1999, pp. 277-303 (hereinafter, "Literature 1") describes an RNA secondary structure prediction method by energy minimalization using the structural modeling based on the tree grammars, and a parsing algorithm.

Elena Rivas and Sean R. Rddy: "The language of RNA: a formal grammar that includes pseudoknots" in *BIOINFORMATICS* vol. 16 no. 4 2000, pp. 334-340 (hereinafter, "Literature 2") describes an RNA secondary structure prediction method by energy minimalization using the structural modeling based on CFGs such as Crossed-interaction grammars having original extension, and a parsing algorithm.

Michael Zuker: *Prediction of RNA Secondary Structure by Energy minimization,* July 8, 1996 (hereinafter, "Literature 3") discloses an Mfold (product name) that is an RNA sequence analysis system using a RNA secondary structure prediction method by Dynamic Programming without using a formal grammar and a parser. According to these literatures, RNA secondary structure prediction accuracy is enhanced by a combination of the scheme such as the use of the formal grammars or the dynamic programming and the energy minimalization scheme.

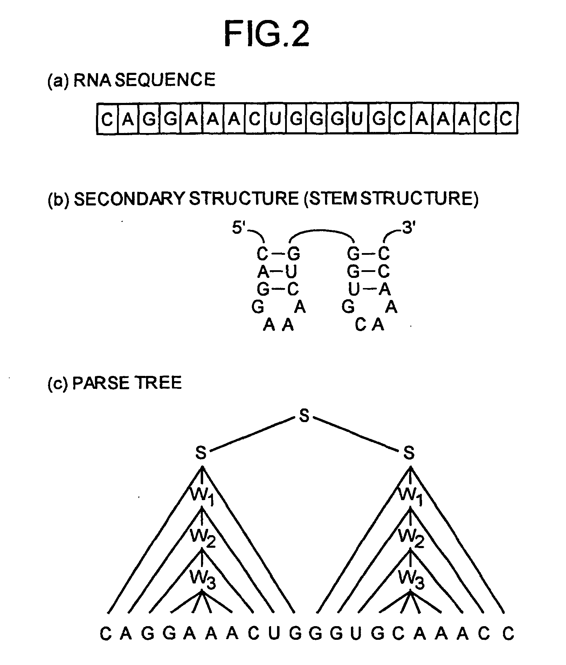

Fig. 2 illustrates an example of a parse tree, according to a conventional art, if the RNA secondary structure has a stem loop. The secondary structure of the RNA sequence illustrated in Fig. 2a is illustrated in Fig. 2b, and the parse tree is illustrated in Fig. 2c. It is noted that a subtree is a fragment of the parse tree having an internal joint as a root. Techniques for performing the secondary structure analysis by creating the parse tree and performing parsing for the structural topology of the RNA secondary structure have been studied, and grammars for principal structural topologies are known.

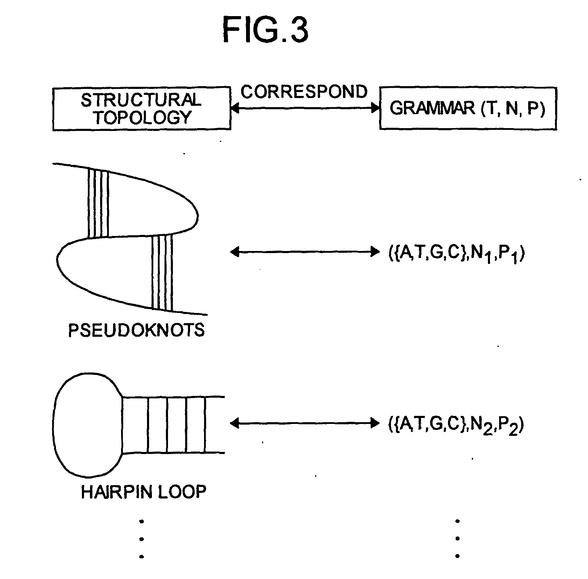

Fig. 3 is a conceptual diagram which illustrates that if a grammar is fixed, a structural topology corresponding to the grammar is specified (vice versa) for the structural topology of the RNA secondary structures, according to the conventional art. A Generative (Formal) grammar (hereinafter, simply a "grammar") includes a finite set of terminal symbols T, a finite set of nonterminal symbols N, and a finite set of production rules P. The set of terminal symbols T includes four symbols of A, T, G, and C for RNA sequences. As illustrated in Fig. 3, the grammar that corresponds to each structural topology can be defined.

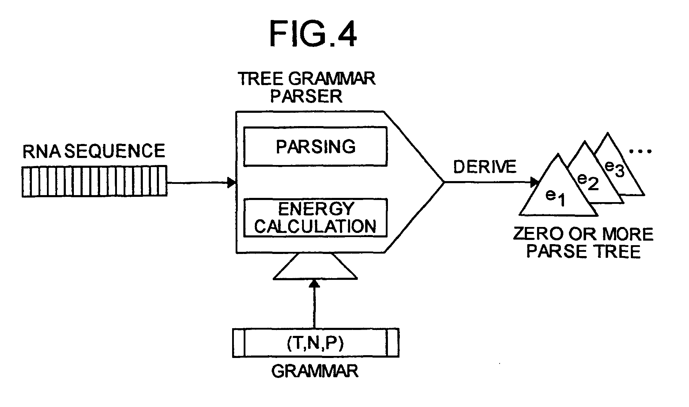

Fig. 4 illustrates an example of deriving the parse tree of an RNA sequence based on known grammar using a conventional tree grammar parser. The RNA sequence of unknown structure is input first to the tree grammar parser. The tree grammar parser has functions including parsing the RNA sequence according to the input known tree grammar and deriving the parse trees, and calculating a sum of free energies of loops, base pairs, and other secondary structure elements for the derived parse trees and of thereby calculating a free energy increments ($\Delta G$) or the like (see Literatures 1 to 3).

In this example, the tree grammar parser does not always derive the parse trees. If the input RNA sequence does not correspond to the grammar (if parsing fails), the parse trees are not input to the tree grammar parser (i.e.,

the number of parse trees is zero). If the tree grammar parser derives multiple parse trees, one parse tree having a minimal free energy obtained as a result of the energy calculation is selected. The tree grammar parser can find a partial structure having the minimal free energy at each step of a derivation process. The tree grammar parser can also output a parse tree having a optimal energy. Thus, the tree grammar parser can realize acceleration and accuracy enhancement by conducting the energy calculation during the parsing process.

Nevertheless, the conventional RNA secondary structure prediction system using the scheme of performing the parsing with energy calculation by means of the tree grammar parser or the like has the following disadvantages. No systematic and efficient method has been proposed for the conventional RNA secondary structure prediction, for integrated management of RNA sequences and extracted grammars, and making the secondary structure prediction more efficiently using the accumulated grammars and RNA sequences.

No systematic and efficient method for searching for an RNA sequence that possibly has a given secondary structure has been proposed.

No systematic and efficient method for easily extracting a secondary structure common to a plurality of RNA sequences has been proposed.

No systematic and efficient method for calculating a similarity based on RNA secondary structures from given RNA sequences has been proposed.

Further, as a method for gene discovery from DNA sequences, there is generally known a method using homology searches, a motif searches or the like. However, the scheme has a disadvantage in that it cannot be used to discover an unknown gene. As explained in the Background Art part, the formal grammar capable of predicting the structural topology of the RNA sequence is obtained. Disadvantageously, however, any gene discovery method using the parse tree derived by the known formal grammar has not yet been proposed.

As explained, the conventional system or the like has many disadvantages. As a result, the conventional system or the like is inferior in convenience and utilization efficiency for both users and a manager of the system or the like.

It is, therefore, scope of the present invention to provide an RNA sequence analyzer, an RNA sequence analysis method, a program, and a recording medium capable of integrally managing RNA sequences and extracted grammars, and making a secondary structure prediction, executing a new analysis scheme or the like more efficiently using the accumulated grammars and RNA sequences.

DISCLOSURE OF THE INVENTION

An RNA sequence analyzer according to one aspect of the present invention includes: a grammar storage unit that stores structural topologies of RNA secondary structures with grammars corresponding to the structural topologies ; a parsing unit that derives parse trees by applying an RNA sequence to the grammars; a goodness-of-fit calculation unit that calculates goodnesses of fit of the parse trees derived by the parsing unit; a sorting unit that sorts the parse trees having the goodnesses of fit that satisfy preset conditions in a descending order of the goodnesses of fit; and an output unit that outputs the parse trees sorted by the sorting unit as secondary structure candidates of the RNA sequence.

According to this analyzer, structural topologies of RNA secondary structures with grammars corresponding to the structural topologies are stored, parse trees are derived by applying an RNA sequence to the grammars, goodnesses of fit of the derived parse trees are calculated, the parse trees having the goodnesses of fit that satisfy preset conditions are sorted in a descending order of the goodnesses of fit, and the sorted parse trees are output as secondary structure candidates of the RNA sequence. Thus, one sequence can be parsed based on multiple grammars. That is, the sequence is subjected to parsing and goodness-of-fit calculation for each parse tree derived from each grammar, thereby obtaining the goodness of fit for each structural topology. As a result, the goodnesses of fit are obtained for the respective grammars, and the grammars can be ranked by sorting the goodnesses of fit. Accordingly, the structural topologies for the grammars can be ranked. Therefore, the structural topologies can be ranked in a descending order of possibility for the given RNA sequence.

An RNA sequence analyzer according to another aspect of the present invention includes: a grammar storage unit that stores a structural topology of RNA secondary structures with a grammar corresponding to the structural topology; a parsing unit that derives parse trees by applying RNA sequences to the grammar; a goodness-of-fit calculation unit that calculates goodnesses of fit of the parse trees derived by the parsing unit; and an output unit that outputs the RNA sequences, from which the parse trees having the goodnesses of fit that satisfy preset conditions are derived, as RNA sequence candidates that could potentially form the secondary structures consistent with the structural topology.

According to this analyzer, a structural topology of RNA secondary structures with a grammar corresponding to the structural topology are stored, parse trees are derived by applying RNA sequences to the grammar, goodnesses of fit of the derived parse trees are calculated, and the RNA sequences, from which the parse trees having the goodnesses of fit that satisfy preset conditions are derived, are output as RNA sequence candidates that could potentially form the secondary structures consistent with the structural topology. Therefore, multiple sequences can be parsed based on one grammar. That is, for a given specific structural topology, a corresponding grammar is obtained. Using the grammar, all of or part of the RNA sequences stored in the RNA sequence database are parsed, respectively, and a group of the RNA sequences which can be successfully parsed with goodnesses of fit that satisfy preset conditions are output as a result. It is thereby possible to search for the RNA sequences that may possibly have the given specific structural topology.

An RNA sequence analyzer according to still another aspect of the present invention includes: a grammar storage unit that stores structural topologies of RNA secondary structures with grammars corresponding to the structural topologies; a parsing unit that derives parse trees by applying RNA sequences to the grammars; a goodness-of-fit calculation unit that calculates goodnesses of fit of the parse trees derived by the parsing unit; an extraction unit that extracts the RNA sequences from which the parse trees having the goodnesses of fit that satisfy preset conditions are derived; and a common structure matrix creation unit that displays the structural topologies and the RNA sequences in a two-dimensional matrix, that gives marks to lattice parts corresponding to the RNA sequences extracted by the extraction unit and the structural topologies in the two-dimensional matrix, and that thereby visualizes the structural topologies common to the RNA sequences.

According to this analyzer, structural topologies of RNA secondary structures with grammars corresponding to the respective structural topologies are stored, parse trees are derived by applying RNA sequences to the grammars, goodnesses of fit of the derived parse trees, the RNA sequences from which the parse trees having the goodnesses of fit that satisfy preset conditions are derived are extracted, the structural topologies and the RNA sequences are displayed in a two-dimensional matrix, marks are given to lattice parts corresponding to the extracted RNA sequences and the structural topologies in the two-dimensional matrix, and the structural topologies common to the RNA sequences are thereby visualized. Hence, it is possible to easily find the structures common to the RNA sequences.

An RNA sequence analyzer according to still another aspect of the present invention includes: a grammar storage unit that stores a structural topology of RNA secondary structures with a grammar corresponding to the structural topology; an RNA sequence production unit that produces RNA sequences transcribed from a DNA sequence input by a user; a parsing unit that derives parse trees by applying the grammar to the RNA sequences produced by the RNA sequence production unit; a goodness-of-fit calculation unit that calculates goodnesses of fit of the parse trees derived by the parsing unit; and a gene prediction unit that predicts parts of the DNA sequence corresponding to the RNA sequences, from which the parse trees having the goodnesses of fit that satisfy preset conditions are derived, as gene candidates.

According to this analyzer, a structural topology of RNA secondary structures with a grammar corresponding to the structural topology are stored, RNA sequences transcribed from a DNA sequence input by a user are produced, parse trees are derived by applying the grammar to the produced RNA sequences, goodnesses of fit of the derived parse trees are calculated, and parts of the DNA sequence corresponding to the RNA sequences, from which the parse trees having the goodnesses of fit that satisfy preset conditions are derived, are predicted as gene candidates. Hence, it is possible to predict that there is a probability that the part of the DNA sequence corresponding to the RNA sequence having a known topology should be a gene.

An RNA sequence analyzer according to still another aspect of the present invention includes: a grammar storage unit that stores a structural topology of RNA secondary structures with a grammar corresponding to the structural topology; a parsing unit that derives parse trees by applying the grammar to RNA sequences; a goodness-of-fit calculation unit that calculates goodnesses of fit of the parse trees derived by the parsing unit; and a similarity calculation unit that calculates a similarity among the RNA sequences based on the goodnesses of fit calculated by the goodness-of-fit calculation unit.

According to this analyzer, a structural topology of RNA secondary structures with a grammar corresponding to the structural topology are stored, parse trees are derived by applying the grammar to RNA sequences, goodnesses of fit of the derived parse trees are calculated, a similarity among the RNA sequences is calculated based on the calculated goodnesses of fit. Hence, it is possible to easily obtain the similarity of the RNA sequences based on the underlying RNA structures.

An RNA sequence analyzer according to still another aspect of the present invention includes: a grammar storage unit that stores structural topologies of RNA secondary structures with grammars corresponding to the structural topologies; a parsing unit that derives parse trees by applying RNA sequences to the grammars; a goodness-of-fit calculation unit that calculates goodnesses of fit of the parse trees derived by the parsing unit; and an extraction unit that extracts the RNA sequences from which the parse trees having the goodnesses of fit that satisfy preset conditions are derived; a goodness-of-fit matrix creation unit that creates a goodness-of-fit matrix which displays the structural topologies and the RNA sequences in a two-dimensional matrix, and which displays the goodnesses of fit on lattice parts corresponding to the RNA sequences extracted by the extraction unit and the structural topologies in the two-dimensional matrix; and a common structure extraction unit that sorts the structural topologies according to the goodnesses of fit for the goodness-of-fit matrix created by the goodness-of-fit matrix creation unit, that parses other RNA sequences based on the grammars corresponding to an order of the sorted structural topologies, and obtains the parse trees having maximum goodnesses of fit, and that extracts the other RNA sequences corresponding to the parse trees having the goodnesses of fit that satisfy the preset conditions.

According to this analyzer, structural topologies of RNA secondary structures with grammars corresponding to the structural topologies are stored, parse trees are derived by applying RNA sequences to the grammars, goodnesses of fit of the derived parse trees are calculated, the RNA sequences from which the parse trees having the goodnesses of fit that satisfy preset conditions are derived are extracted, a goodness-of-fit matrix which displays the structural topologies and the RNA sequences in a two-dimensional matrix, and which displays the goodnesses of fit on lattice parts corresponding to the extracted RNA sequences and the structural topologies in the two-dimensional matrix, is created, the structural topologies are sorted according to the goodnesses of fit for the goodness-of-fit matrix, other RNA sequences are parsed based on the grammar corresponding to an order of the sorted structural topologies, the parse trees having optimum goodnesses of fit are obtained, and the other RNA sequences corresponding to the parse trees having the goodnesses of fit that satisfy the preset conditions are extracted. Hence, it is possible to easily find the RNA sequences having the common structure.

An RNA sequence analysis method according to one aspect of the present invention includes: a grammar storage step that stores structural topologies of RNA secondary structures with grammars corresponding to the structural topologies; a parsing step that derives parse trees by applying an RNA sequence to the grammars; a goodness-of-fit calculation step that calculates goodnesses of fit of the parse trees derived by the parsing step; a sorting step that sorts the parse trees having the goodnesses of fit that satisfy preset conditions in a descending order of the goodnesses of fit; and an output step that outputs the parse trees sorted by the sorting step as secondary structure candidates of the RNA sequence.

According to this analysis method, structural topologies of RNA secondary structures with grammars corresponding to the structural topologies are stored, parse trees are derived by applying an RNA sequence to the grammars, goodnesses of fit of the derived parse trees are calculated, the parse trees having the goodnesses of fit that satisfy preset conditions are sorted in a descending order of the goodnesses of fit, and the sorted parse trees are output as secondary structure candidates of the RNA sequence. Thus, one sequence can be parsed based on multiple grammars. That is, the sequence is subjected to parsing and goodness-of-fit calculation for each parse tree derived from each grammar, thereby obtaining the goodness of fit for each structural topology. As a result, the goodnesses of fit are obtained for the respective grammars, and the grammars can be ranked by sorting the goodnesses of fit. Accordingly, the structural topologies for the grammars can be ranked. Therefore, the structural topologies can be ranked in a descending order of possibility for the given RNA sequence.

An RNA sequence analysis method according to another aspect of the present invention includes: a grammar storage step that stores a structural topology of RNA secondary structures with a grammar corresponding to the structural topology; a parsing step that derives parse trees by applying RNA sequences to the grammar; a goodness-of-fit calculation step that calculates goodnesses of fit of the parse trees derived by the parsing step; and an output step that outputs the RNA sequences, from which the parse trees having the goodnesses of fit that satisfy preset conditions are derived, as RNA sequence candidates that could potentially form the secondary structures consistent with the structural topology.

According to this analysis method, a structural topology of RNA secondary structures with a grammar corresponding to the structural topology are stored, parse trees are derived by applying RNA sequences to the grammar, goodnesses of fit of the derived parse trees are calculated, and the RNA sequences, from which the parse trees having the goodnesses of fit that satisfy preset conditions are derived, are output as RNA sequence candidates that could potentially form the secondary structures consistent with the structural topology. Therefore, multiple sequences can be parsed based on one grammar. That is, for a given specific structural topology, a corresponding grammar is obtained. Using the grammar, all of or part of the RNA sequences stored in the RNA sequence database are parsed, respectively, and a group of the RNA sequences which can be successfully parsed with goodnesses of fit that satisfy preset conditions are output as a result. It is thereby possible to search for the RNA sequences that may possibly have the given specific structural topology.

An RNA sequence analysis method according to still another aspect of the present invention includes: a grammar storage step that stores structural topologies of RNA secondary structures with grammars corresponding to the structural topologies; a parsing step that derives parse trees by applying RNA sequences to the grammars; a goodness-of-fit calculation step that calculates goodnesses of fit of the parse trees derived by the parsing step; an extraction step that extracts the RNA sequences from which the parse trees having the goodnesses of fit that satisfy preset conditions are derived; and a common structure matrix creation step that displays the structural topologies and the RNA sequences in a two-dimensional matrix, that gives marks to lattice parts corresponding to the RNA sequences extracted by the extraction step and the structural topologies in the two-dimensional matrix, and that thereby visualizes the structural topologies common to the RNA sequences.

According to this analysis method, structural topologies of RNA secondary structures with grammars corresponding to the respective structural topologies are stored, parse trees are derived by applying RNA sequences to the grammars, goodnesses of fit of the derived parse trees, the RNA sequences from which the parse trees having the goodnesses of fit that satisfy preset conditions are derived are extracted, the structural topologies and the RNA sequences are displayed in a two-dimensional matrix, marks are given to lattice parts corresponding to the extracted RNA sequences and the structural topologies in the two-dimensional matrix, and the structural topologies common to the RNA sequences are thereby visualized. Hence, it is possible to easily find the structures common to the RNA sequences.

An RNA sequence analysis method according to still another aspect of the present invention includes: a grammar storage step that stores a structural topology of RNA secondary structures with a grammar corresponding to the structural topology; an RNA sequence production step that produces RNA sequences transcribed from a DNA sequence input by a user; a parsing step that derives parse trees by applying the grammar to the RNA sequences produced by the RNA sequence production step; a goodness-of-fit calculation step that calculates goodnesses of fit of the parse trees derived by the parsing step; and a gene prediction step that predicts parts of the DNA sequence corresponding to the RNA sequences, from which the parse trees having the goodness of fit that satisfy preset conditions are derived, as gene candidates.

According to this analysis method, a structural topology of RNA secondary structures with a grammar corresponding to the structural topology are stored, RNA sequences transcribed from a DNA sequence input by a user are produced, parse trees are derived by applying the grammar to the produced RNA sequences, goodnesses of fit of the derived parse trees are calculated, and parts of the DNA sequence corresponding to the RNA sequences, from which the parse trees having the goodnesses of fit that satisfy preset conditions are derived, are predicted as gene candidates. Hence, it is possible to predict that there is a probability that the part of the DNA sequence corresponding to the RNA sequence having known topology should be a gene part.

An RNA sequence analysis method according to still another aspect of the present invention includes: a grammar storage step that stores a structural topology of RNA secondary structures with a grammar corresponding to the structural topology; a parsing step that derives parse trees by applying the grammar to RNA sequences; a goodness-of-fit calculation step that calculates goodnesses of fit of the parse trees derived by the parsing step; and a similarity calculation step that calculates a similarity among the RNA sequences based on the goodnesses of fit calculated by the goodness-of-fit calculation step.

According to this analysis method, a structural topology of RNA secondary structures with a grammar corresponding to the structural topology are stored, parse trees are derived by applying the grammar to RNA sequences, goodnesses of fit of the derived parse trees are calculated, a similarity among the RNA sequences is calculated based on the calculated goodnesses of fit. Hence, it is possible to easily obtain the similarity of the RNA sequences based on the underlying RNA structures.

An RNA sequence analysis method according to still another aspect of the present invention includes: a grammar storage step that stores structural topologies of RNA secondary structures with grammars corresponding to the structural topologies; a parsing step that derives parse trees by applying RNA sequences to the grammars; a goodness-of-fit calculation step that calculates goodnesses of fit of the parse trees derived by the parsing step; and an extraction step that extracts the RNA sequences from which the parse trees having the goodnesses of fit that satisfy preset conditions are derived; a goodness-of-fit matrix creation step that creates a goodness-of-fit matrix which displays the structural topologies and the RNA sequences in a two-dimensional matrix, and which displays the goodnesses of fit on lattice parts corresponding to the RNA sequences extracted by the extraction step and the structural topologies in the two-dimensional matrix; and a common structure extraction step that sorts the structural topologies according to the goodnesses of fit for the goodness-of-fit matrix created by the goodness-of-fit matrix creation step, that parses other RNA sequences based on the grammar corresponding to an order of the sorted structural topologies, and obtains the parse trees having optimum goodnesses of fit, and that extracts the other RNA sequences corresponding to the parse trees having the goodnesses of fit that satisfy the preset conditions.

According to this analysis method, structural topologies of RNA secondary structures with grammars corresponding to the structural topologies are stored, parse trees are derived by applying RNA sequences to the grammars, goodnesses of fit of the derived parse trees are calculated, the RNA sequences from which the parse trees having the goodnesses of fit that satisfy preset conditions are derived are extracted, a goodness-of-fit matrix which displays the structural topologies and the RNA sequences in a two-dimensional matrix, and which displays the goodnesses of fit on lattice parts corresponding to the extracted RNA sequences and the structural topologies in the two-dimensional matrix, is created, the structural topologies are sorted according to the goodnesses of fit for the goodness-of-fit matrix, other RNA sequences are parsed based on the grammar corresponding to an order of the sorted structural topologies, the parse trees having optimum goodnesses of fit are obtained, and the other RNA sequences corresponding to the parse trees having the goodnesses of fit that satisfy the preset conditions are extracted. Hence, it is possible to easily find the RNA sequences having the common structure.

A computer program that makes a computer to execute an RNA sequence analysis method according to one aspect of the present invention includes: a grammar storage step that stores structural topologies of RNA secondary structures with grammars corresponding to the structural topologies; a parsing step that derives parse trees by applying an RNA sequence to the grammars; a goodness-of-fit calculation step that calculates goodnesses of fit of the parse trees derived by the parsing step; a sorting step that sorts the parse trees having the goodnesses of fit that satisfy preset conditions in a descending order of the goodnesses of fit; and an output step that outputs the parse trees sorted by the sorting step as secondary structure candidates of the RNA sequence.

According to this program, structural topologies of RNA secondary structures with grammars corresponding to the structural topologies are stored, parse trees are derived by applying an RNA sequence to the grammars, goodnesses of fit of the derived parse trees are calculated, the parse trees having the goodnesses of fit that satisfy preset conditions are sorted in a descending order of the goodnesses of fit, and the sorted parse trees are output as secondary structure candidates of the RNA sequence. Thus, one sequence can be parsed based on multiple grammars. That is, the sequence is subjected to parsing and goodness-of-fit calculation for each parse tree derived from each grammar, thereby obtaining the goodness of fit for each structural topology. As a result, the goodnesses of fit are obtained for the respective grammars, and the grammars can be ranked by sorting the goodnesses of fit. Accordingly, the structural topologies for the grammars can be ranked. Therefore, the structural topologies can be ranked in a descending order of possibility for the given RNA sequence.

A computer program that makes a computer to execute an RNA sequence analysis method according to another aspect of the present invention includes: a grammar storage step that stores a structural topology of RNA secondary structures with a grammar corresponding to the structural topology; a parsing step that derives parse trees by applying RNA sequences to the grammar; a goodness-of-fit calculation step that calculates goodnesses of fit of the parse trees derived by the parsing step; and an output step that outputs the RNA sequences, from which the parse trees having the goodnesses of fit that satisfy preset conditions are derived, as RNA sequence candidates that could potentially form the secondary structures consistent with the structural topology.

According to this program, a structural topology of RNA secondary structures with a grammar corresponding to the structural topology are stored, parse trees are derived by applying RNA sequences to the grammar, goodnesses of fit of the derived parse trees are calculated, and the RNA sequences, from which the parse trees having the goodnesses of fit that satisfy preset conditions are derived, are output as RNA sequence candidates that could potentially form the secondary structures consistent with the structural topology. Therefore, multiple sequences can be parsed based on one grammar. That is, for a given specific structural topology, a corresponding grammar is obtained. Using the grammar, all of or part of the RNA sequences stored in the RNA sequence database are parsed, respectively, and a group of the RNA sequences which can be successfully parsed with goodnesses of fit that satisfy preset conditions are output as a result. It is thereby possible to search for the RNA sequences that may possibly have the given specific structural topology.

A computer program that makes a computer to execute an RNA sequence analysis method according to still another aspect of the present invention includes: a grammar storage step that stores structural topologies of RNA secondary structures with grammars corresponding to the structural topologies; a parsing step that derives parse trees by applying RNA sequences to the grammars; a goodness-of-fit calculation step that calculates goodnesses of fit of the parse trees derived by the parsing step; an extraction step that extracts the RNA sequences from which the parse trees having the goodnesses of fit that satisfy preset conditions are derived; and a common structure matrix creation step that displays the structural topologies and the RNA sequences in a two-dimensional matrix, that gives marks to lattice parts corresponding to the RNA sequences extracted by the extraction step and the structural topologies in the two-dimensional matrix, and that thereby visualizes the structural topologies common to the RNA sequences.

According to this program, structural topologies of RNA secondary structures with grammars corresponding to the respective structural topologies are stored, parse trees are derived by applying RNA sequences to the grammars, goodnesses of fit of the derived parse trees, the RNA sequences from which the parse trees having the goodnesses of fit that satisfy preset conditions are derived are extracted, the structural topologies and the RNA sequences are displayed in a two-dimensional matrix, marks are given to lattice parts corresponding to the extracted RNA sequences and the structural topologies in the two-dimensional matrix, and the structural topologies common to the RNA sequences are thereby visualized. Hence, it is possible to easily find the structures common to the RNA sequences.

A computer program that makes a computer to execute an RNA sequence analysis method according to still another aspect of the present invention includes: a grammar storage step that stores a structural topology of RNA secondary structures with a grammar corresponding to the structural topology; an RNA sequence production step that produces RNA sequences transcribed from a DNA sequence input by a user; a parsing step that derives parse trees by applying the grammar to the RNA sequences produced by the RNA sequence production step; a goodness-of-fit calculation step that calculates goodnesses of fit of the parse trees derived by the parsing step; and a gene prediction step that predicts parts of the DNA sequence corresponding to the RNA sequences, from which the parse trees having the goodnesses of fit that satisfy preset conditions are derived, as gene candidates.

According to this program, a structural topology of RNA secondary structures with a grammar corresponding to the structural topology are stored, RNA sequences transcribed from a DNA sequence input by a user are produced, parse trees are derived by applying the grammar to the produced RNA sequences, goodnesses of fit of the derived parse trees are calculated, and parts of the DNA sequence corresponding to the RNA sequences, from which the parse trees having the goodnesses of fit that satisfy preset conditions are derived, are predicted as gene candidates. Hence, it is possible to predict that there is a probability that the part of the DNA sequence corresponding to the RNA sequence having a known topology should be a gene.

A computer program that makes a computer to execute an RNA sequence analysis method according to still another aspect of the present invention includes: a grammar storage step that stores a structural topology of RNA secondary structures with a grammar corresponding to the structural topology; a parsing step that derives parse trees by applying the grammar to RNA sequences; a goodness-of-fit calculation step that calculates goodnesses of fit of the parse trees derived by the parsing step; and a similarity calculation step that calculates a similarity among the RNA sequences based on the goodnesses of fit calculated by the goodness-of-fit calculation step.

According to this program, a structural topology of an RNA secondary structures with a grammar corresponding to the structural topology are stored, parse trees are derived by applying the grammar to RNA sequences, goodnesses of fit of the derived parse trees are calculated, a similarity among the RNA sequences is calculated based on the calculated goodnesses of fit. Hence, it is possible to easily obtain the similarity of the RNA sequences based on the underlying RNA structures.

A computer program that makes a computer to execute an RNA sequence analysis method according to still another aspect of the present invention includes: a grammar storage step that stores structural topologies of RNA secondary structures with grammars corresponding to the structural topologies; a parsing step that derives parse trees by applying RNA sequences to the grammars; a goodness-of-fit calculation step that calculates goodnesses of fit of the parse trees derived by the parsing step; and an extraction step that extracts the RNA sequences from which the parse trees having the goodnesses of fit that satisfy preset conditions are derived; a goodness-of-fit matrix creation step that creates a goodness-of-fit matrix which displays the structural topologies and the RNA sequences in a two-dimensional matrix, and which displays the goodnesses of fit on lattice parts corresponding to the RNA sequences extracted by the extraction step and the structural topologies in the two-dimensional matrix; and a common structure extraction step that sorts the structural topologies according to the goodnesses of fit for the goodness-of-fit matrix created by the goodness-of-fit matrix creation step, that parses other RNA sequences based on the grammars corresponding to an order of the sorted structural topologies, and obtains the parse trees having maximum goodnesses of fit, and that extracts the other RNA sequences corresponding to the parse trees having the goodnesses of fit that satisfy the preset conditions.

According to this program, structural topologies of RNA secondary structures with grammars corresponding to the structural topologies are stored, parse trees are derived by applying RNA sequences to the grammars, goodnesses of fit of the derived parse trees are calculated, the RNA sequences from which the parse trees having the goodnesses of fit that satisfy preset conditions are derived are extracted, a goodness-of-fit matrix which displays the structural topologies and the RNA sequences in a two-dimensional matrix, and which displays the goodnesses of fit on lattice parts corresponding to the extracted RNA sequences and the structural topologies in the two-dimensional matrix, is created, the structural topologies are sorted according to the goodnesses of fit for the goodness-of-fit matrix, other RNA sequences are parsed based on the grammar corresponding to an order of the sorted structural topologies, the parse trees having optimum goodnesses of fit are obtained, and the other RNA sequences corresponding to the parse trees having the goodnesses of fit that satisfy the preset conditions are extracted. Hence, it is possible to easily find the RNA sequences having the common structure.

Furthermore, the present invention relates to the recording medium. The recording medium according to the present invention records the program described above.

This recording medium can realize the program using a computer by making the computer read the program recorded on the recording medium, and exhibit the same advantages as those of each program.

BRIEF DESCRIPTION OF THE DRAWINGS

Fig. 1 is an illustration for explaining examples of structural topology of RNA secondary structures; Fig. 2 illustrates one example of a parse tree corresponding to an RNA secondary structure having stem-loops, according to the conventional art; Fig. 3 is a conceptual diagram which illustrates that if a grammar is fixed, a corresponding structural topology is specified for the structural topology of the RNA secondary structures according to the conventional art; Fig. 4 illustrates one example of deriving a parse tree of an RNA sequence by a given grammar, Fig. 5 is a block diagram which illustrate one example of the configuration of a system to which the present invention is applied; Fig. 6 illustrates one example of information stored in a grammar database 106b; Fig. 7 is a conceptual diagram which illustrates one example of an RNA secondary structure prediction process performed by the system according to one embodiment;

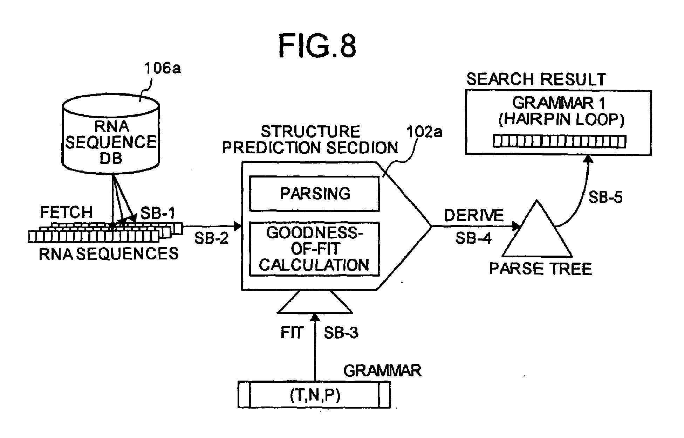

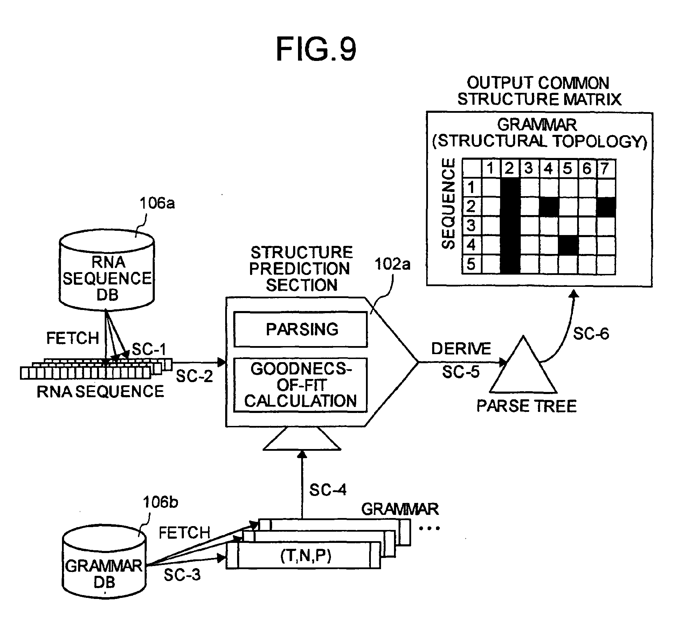

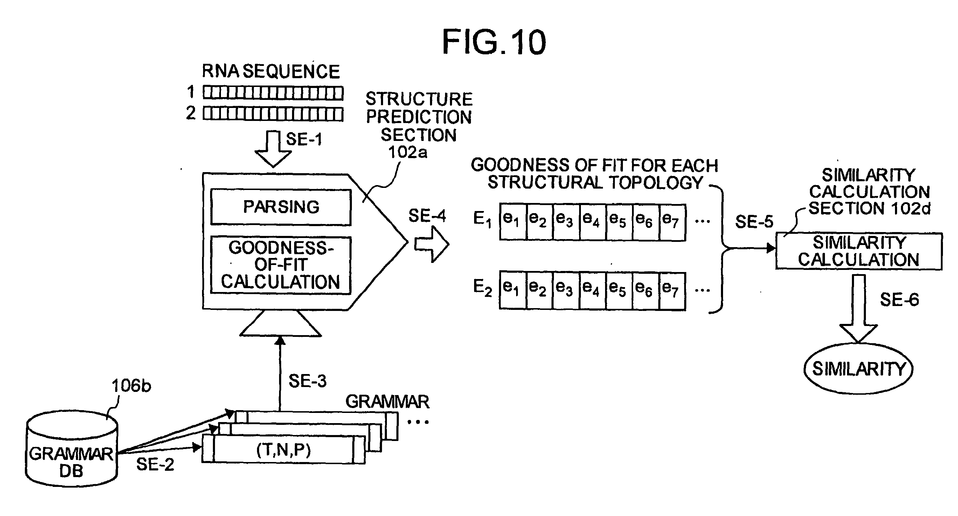

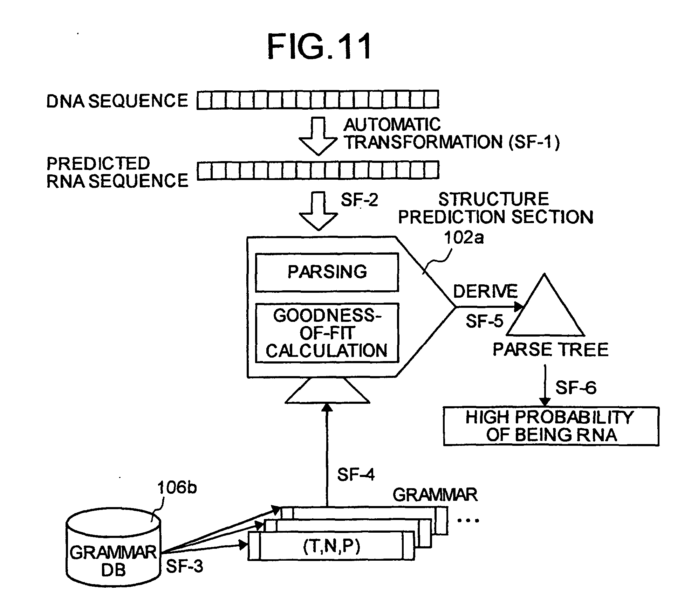

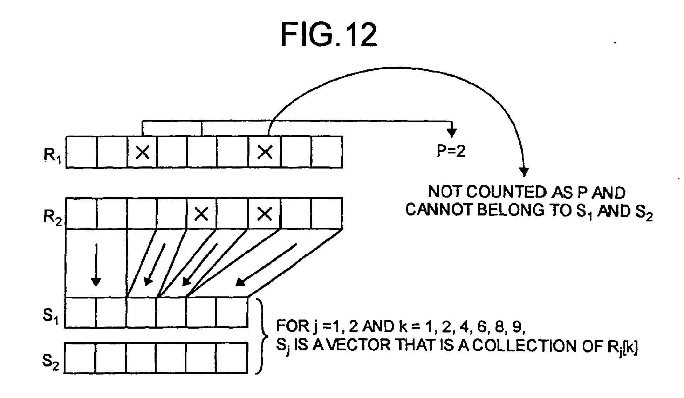

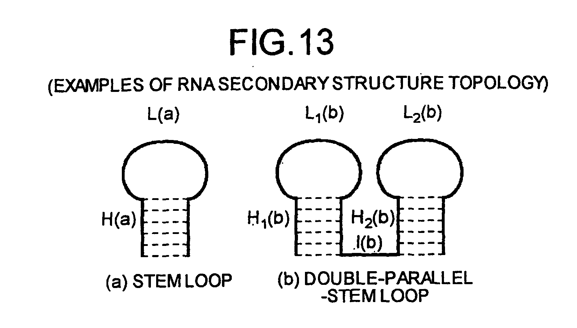

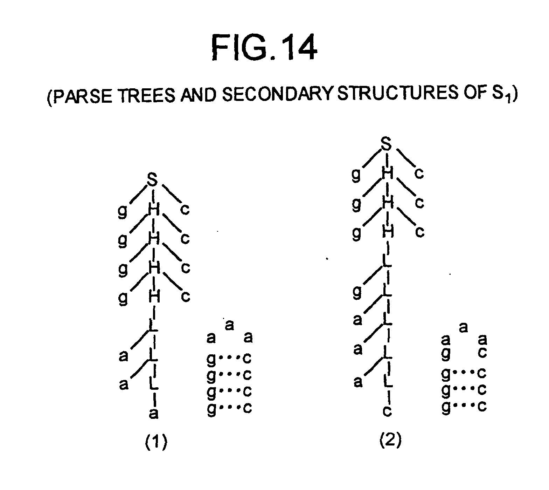

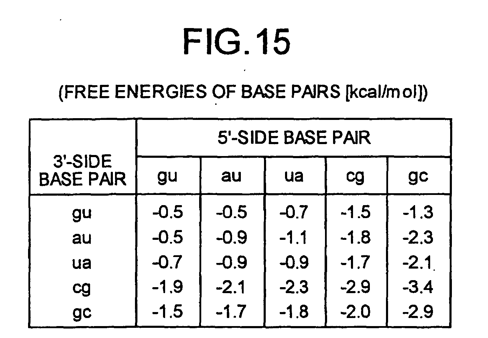

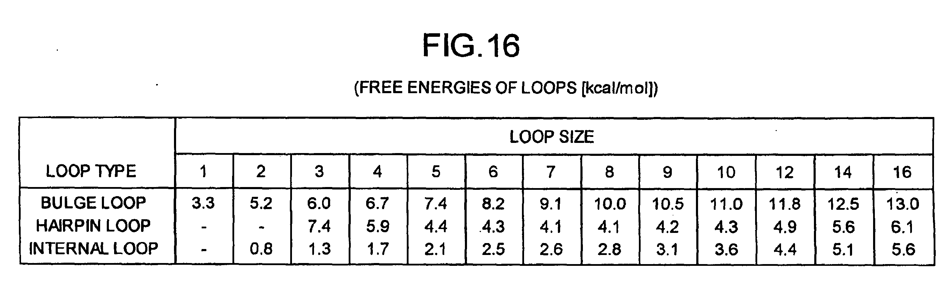

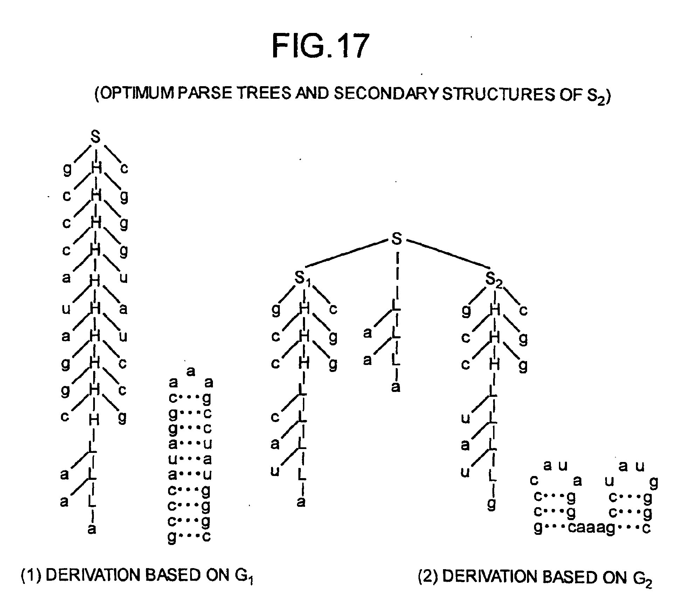

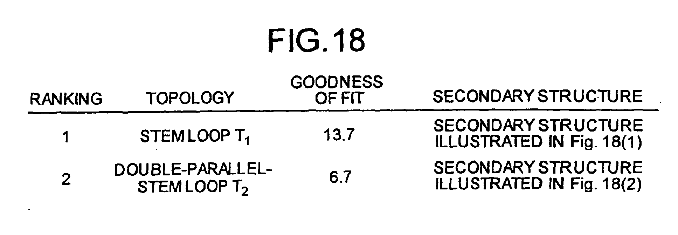

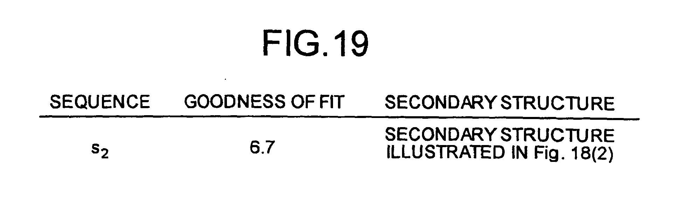

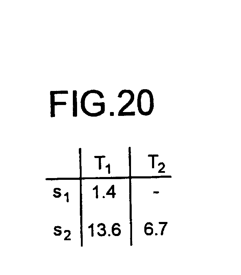

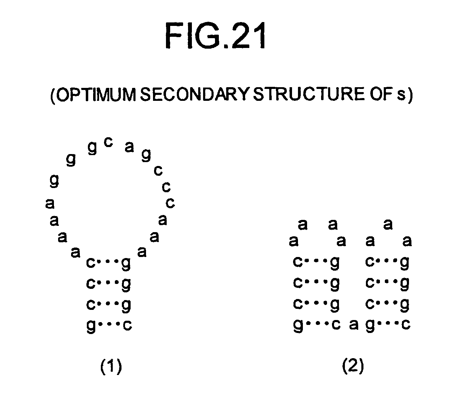

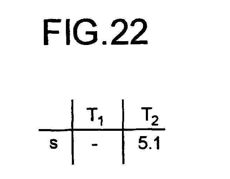

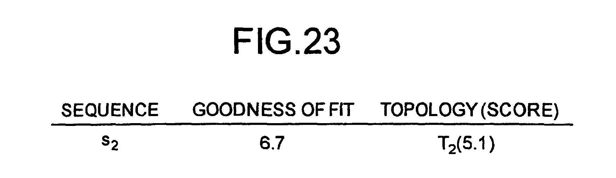

Fig. 8 is a conceptual diagram which illustrates one example of RNA sequences of similar structures extraction process performed by the system according to the embodiment; Fig. 9 is a conceptual diagram which illustrates one example of a common structure extraction process performed by the system according to the present invention; Fig. 10 is a conceptual diagram which illustrates one example of a structure similarity calculation process performed by the system according to the present invention; Fig. 11 is a conceptual diagram which illustrates one example of a gene prediction process performed by the system according to the embodiment; Fig. 12 is an illustration for explaining the concept of a penalty P and similarity vectors $s_1$ and $s_2$; Fig. 13 illustrates examples of RNA secondary structure topology; Fig. 14 illustrates a parse tree and a secondary structure of $s_1$; Fig. 15 illustrates free energies of base pairs; Fig. 16 illustrates free energies of loops; Fig. 17 illustrates an optimum parse tree and a corresponding secondary structure according to a goodness-of-fit index of $-\Delta G$ for each grammar, Fig. 18 illustrates structure candidates fit to $s_2$ in a selected topology group; Fig. 19 illustrates a sequence candidate which may possibly have a selected topology; Fig. 20 illustrates a matrix having goodnesses of fit of parse trees as elements; Fig. 21 illustrates an optimum secondary structure of s; Fig. 22 illustrates a matrix having the goodnesses of fit of parse trees as an element; and Fig. 23 illustrates one example of an output result.

### BEST MODE FOR CARRYING OUT THE INVENTION

Exemplary embodiments of an RNA sequence analyzer, an RNA sequence analysis method, a program, and a recording medium according to the present invention will be explained hereinafter in detail with reference to the accompanying drawings. It should be noted that the present invention is not limited to the embodiments.

Particularity in the embodiments, the present invention is explained by examples that employ tree grammars as structure modeling grammars. However, the present invention is not limited to the examples and it should be noted that any formal grammars can be similarly applied.

[Outline of System]

The outline of a system according to the present invention will be explained first, followed by the configuration, procedures, and the like of the system.

Schematically, this system has the following basic features. An RNA sequence analyzer in this system stores structural topologies of RNA secondary structures with grammars corresponding to the structural topologies, derives parse trees by applying the RNA sequence to the grammars; calculates goodnesses of fit of the respective derived parse trees, sorts the parse trees having the goodnesses of fit that satisfy preset conditions in a descending order of the goodnesses of fit, and outputs the sorted parse trees as secondary structure candidates of the RNA sequence. Examples of the formal grammars include tree grammars, context-free grammars, and the like. Since the tree grammars are quite feasible for modeling pseudoknots, it is preferable to use the tree grammars as the modeling grammars for RNA secondary structures.

The RNA sequence analyzer according to the present invention calculates goodnesses of fit of the derived parse trees, and outputs RNA sequences, from which the parse trees having the goodness of fit that satisfy preset conditions are derived, as RNA sequence candidates that could potentially form the secondary structures consistent with the structural topology.

Further, the RNA sequence analyzer derives parse trees from which parse trees having goodnesses of fit that satisfy preset conditions are derived, displays each structural topology and each RNA sequence in a two-dimensional matrix, gives marks to lattice parts corresponding to the extracted RNA sequences and structural topologies in the two-dimensional matrix, and thereby visualizes structural topologies common to the RNA sequences.

The RNA sequence analyzer according to the present invention, produces RNA sequences transcribed from a DNA sequence input by a user, derives parse trees by applying the grammar to the RNA sequences, calculates goodnesses of fit of the derived parse trees; and predicts, as gene candidates, DNA sequence parts corresponding to the RNA sequences from which the parse trees having the goodnesses of fit that satisfy preset conditions are derived.

Moreover, the analyzer according to the present invention stores structural topologies of RNA secondary structures with grammars corresponding to the structural topologies, derives parse trees by applying the grammars to RNA sequences, calculates goodnesses of fit of the derived parse trees, and calculates a similarity among the RNA sequences based on the calculated goodnesses of fit for the underlying RNA structures.

[System Configuration]

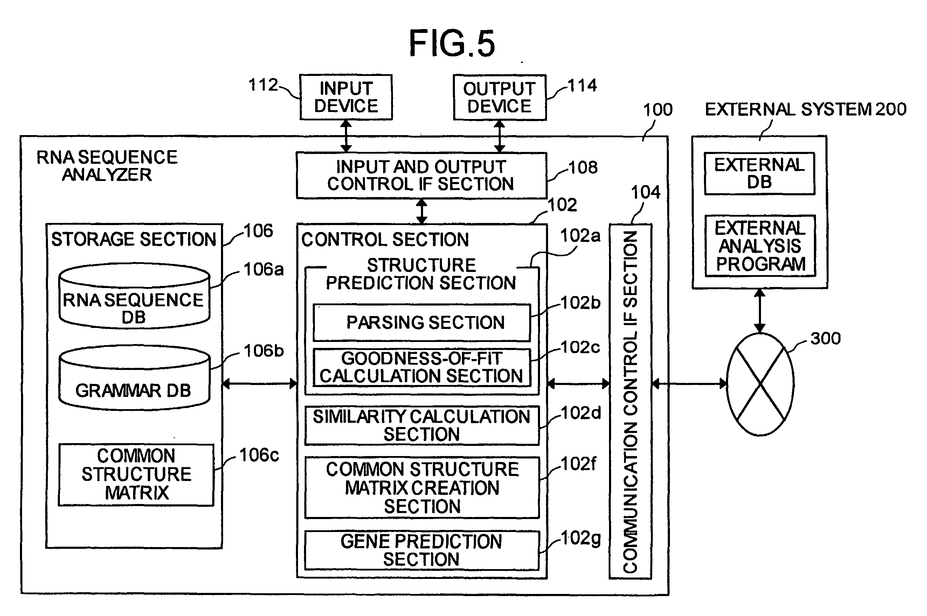

The configuration of the system will first be explained. Fig. 5 is a block diagram which illustrates one example of the configuration of the system to which the present invention is applied. In Fig. 5, only sections related to the present invention in the configuration are conceptually illustrated. The system is schematically constituted so that an RNA sequence analyzer 100 that is an RNA sequence analyzer for analyzing sequence information, and an external system 200 that provides external databases related to the sequence information and the like, external analysis programs for homology searches, and the like are communicably connected to each other through a network 300.

In Fig. 5, the network 300 functions to connect the RNA sequence analyzer 100 and the external system 200 to each other, and is, for example, the Internet.

In Fig. 5, the external system 200 and the RNA sequence analyzer are connected to each other via the network 300, and the external system 200 functions to provide a website that executes external analysis programs for users. The programs include external databases related to sequence information, homology searches, and motif searches.

In Fig. 5, the external system 200 may be constituted as a WEB server, an ASP server, or the like, and hardware of the external system 200 may include an information processing apparatus such as a commercially available workstation or personal computer, and accessories of the apparatus. Respective functions of the external system 200 are realized by a CPU, a disk device, a memory device, an input device, an output device, a communication control device, and the like in the hardware configuration of the external system 200 as well as programs for controlling these devices, and the like.

In Fig. 5, the RNA sequence analyzer 100 schematically includes a control section 102 such as the CPU for generally controlling entirety of the RNA sequence analyzer 100, a communication control interface section 104 connected to a communication device (not illustrated) such as a router connected to a communication line or the like, an input and output control interface 108 connected to the input device 112 and the output device 114, and a storage section 106 that stores various databases and tables (an RNA sequence database 106a to a common structure matrix 106c). The respective sections are communicably connected to one another through arbitrary communication lines. In addition, this RNA sequence analyzer 100 is communicably connected to the network 300 through the communication device such as the router and a wired or wireless communication line such as a dedicated line.

The various databases stored in the storage section 106 (the RNA sequence database 106a to the common structure matrix 106c) are storage units for a hard disk device or the like, and store various programs, tables, files, databases, webpage files, and the like used for various processings.

Among the constituent elements of the storage section 106, the RNA sequence database 106a is a database that stores RNA sequences. The RNA sequence database 106a may be an external RNA sequence database accessed through the internet, or an in-house database created by copying the database, by storing original sequence information, or by adding individual annotation information and the like to the database. The RNA sequence database 106a may store RNA sequences produced in advance based on a DNA sequence database for cDNAs or the like or RNA sequences dynamically produced as needed.

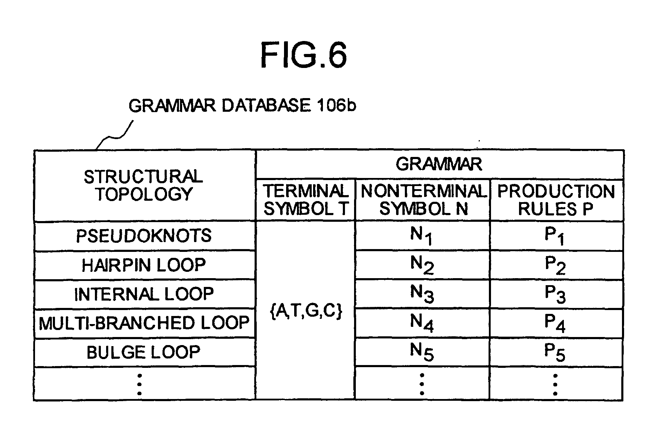

A grammar database 106b is a grammar storage unit that stores structural topologies of RNA secondary structures with grammars corresponding to the respective structural topologies. Fig. 6 illustrates one example of information stored in the grammar database 106b. As illustrated in Fig. 6, the grammar database 106b stores the structural topologies with the grammars corresponding to the respective topologies. As illustrated in Fig. 6, the grammar database 106b may store the structural topologies and the grammars in a one-to-one correspondence. Alternatively, the grammar corresponding to complex topologies (e.g., topologies each having both pseudoknots and a hairpin loop), the grammar for RNA having a characteristic structure (e.g., a structural topology characteristic of rRNA), the grammar for a topology common to RNAs in a predetermined category, and the grammar corresponding to all possible RNA topologies may be even specified.

The common structure matrix 106c is a table (storage area) for displaying the structural topologies and the RNA sequences in a two-dimensional matrix.

In Fig. 5, the communication control interface 104 controls the communication between the RNA sequence analyzer 100 and the network 300 (or the communication device such as the router). Namely, the communication control interface 104 functions to communicate data with other terminals through communication lines.

In Fig. 5, the input and output control interface section 108 controls the input device 112 and the output device 114. As the output device 114, a monitor (including a home television set), a loudspeaker or the like can be used (it is noted that the output device 114 is sometimes referred to as "monitor" hereinafter). As the input device 112, a keyboard, a mouse, a microphone, or the like can be used. The monitor also realizes a pointing device function in cooperation with the mouse.

In Fig. 5, the control section 102 includes an internal memory for storing various programs such as an OS (Operating System), programs for specifying various processing procedures, and required data. Using these programs and the like, information processings for executing various processings are performed. The control section 102 functionally conceptually includes a structure prediction section 102a, a similarity calculation section 102d, a common structure matrix creation section 102f, and a gene prediction section 102g.

Among these sections, the structure prediction section 102a has a function (a parsing section 102b) that performs parsing for the RNA sequences according to input grammars, and that derives parse trees, a function (a goodness-of-fit calculation section 102c) that calculates goodnesses of fit of the respective derived parse trees, and the like.

The similarity calculation section 102d is a similarity calculation unit that calculates a similarity among RNA sequences.

The common structure matrix creation section 102f is an extraction unit that extracts RNA sequences from which the parse trees having goodnesses of fit that satisfy preset conditions are derived, a common structure matrix creation unit that displays the structural topologies and the RNA sequences in the two-dimensional matrix, that gives marks to lattice parts corresponding to the RNA sequences extracted by the extraction units and the structural topologies in the two-dimensional matrix, and that thereby visualizes structural topologies common to the RNA sequences, a goodness-of-fit matrix creation unit that creates a goodness-of-fit matrix for displaying the goodnesses of fit in the lattice parts corresponding to the RNA sequences extracted by the extraction unit and the structural topologies in the two-dimensional matrix, and a common structure extraction unit that sorts the structural topologies according to the goodnesses of fit for the goodness-of-fit matrix created by the goodness-of-fit creation unit, that performs parsing for the other RNA sequences based on the grammar corresponding to an order of the sorted structural topologies, that obtains a parse tree having the optimum goodness of fit, and that extracts the other RNA sequences corresponding to the parse trees having the goodnesses of fit satisfying the preset conditions.

The gene prediction section 102g is an RNA sequence production unit that produces RNA sequences transcribed from a DNA sequence input by a user, and a gene prediction unit that predicts, as gene candidates, DNA sequence parts corresponding to the RNA sequences from which the parse trees having the goodnesses of fit satisfying the preset conditions are derived. Details of processings performed by these sections will be explained later.

[System Processings]

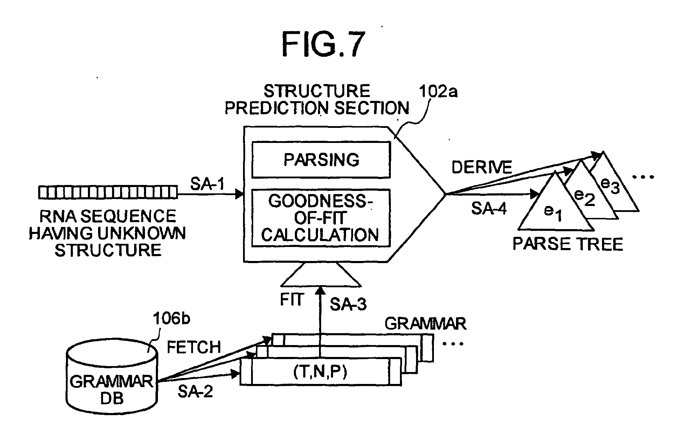

One example of processings performed by the system according to the embodiment constituted as explained above will next be explained with reference to Figs. 7 to 11.

[RNA Secondary Structure Prediction Processing]

The detail of an RNA secondary structure prediction processing will first be explained with reference to Fig. 7. Fig. 7 is a conceptual diagram which illustrates one example of the RNA secondary structure prediction performed by the system according to the embodiment.

Grammars that represent known RNA structural topologies are first accumulated in the grammar database 106b. The user inputs an RNA sequence, the structure of which is unknown and the secondary structure of which the user is to specify, to the RNA sequence analyzer 100 through the input device 112 (at a step SA-1). If so, the structure prediction section 102a fetches grammars from the grammar database 106b by a processing performed by the parsing section 102b (at a step SA-2), and parses the RNA sequence by applying the respective grammars to the RNA sequence (at a step SA-3). The user may input the RNA sequence by selecting a desired sequence from the RNA sequence database 106a, by selecting the desired sequence from the external database in the external system 200, or by directly inputting the desired sequence.

The structure prediction section 102a calculates goodnesses of fit of the respective parse trees that is obtained as a result of successful parsing and that is derived, based on a free energy increments (ΔG) obtained by, for example, calculating a sum of free energies of loops, base pairs, and the other secondary structure elements, or the like, by a processing performed by the goodness-of-fit calculation section 102c. As the goodness-of-fit calculation method, one of the methods disclosed in Literatures 1 to 3 and the conventional methods may be used.

The structure prediction section 102a sorts the parse trees having the goodnesses of fit that satisfy the preset conditions in a descending order of goodness of fit (at a step SA-4).

The structure prediction section 102a outputs the sorted parse trees and the goodnesses of fit of the parse trees to the output device 114 through the input and output control interface section 108. Thus, one sequence input by the user can be parsed based on multiple grammars. That is, the sequence is subjected to parsing and goodness-of-fit calculation for each grammar, thereby obtaining the goodness of fit. As a result, goodnesses of fit corresponding to the respective grammars can be obtained. The grammars can be ranked by sorting the goodnesses of fit. Accordingly, the structural topologies for the grammars can be ranked, thereby making it possible to see the structural topologies in a descending order of possibility for the given RNA sequence. The RNA secondary structure prediction processing is thereby finished.

[RNA Sequences of Similar Structures Extraction Processing]

The detail of RNA sequences of similar structures extraction processing will be explained with reference to Fig. 8. Fig. 8 is a conceptual diagram which illustrates one example of RNA sequences of possible common structures extraction process performed by the system according to the embodiment.

The user selects a grammar corresponding to a specific structural topology from the grammar database 106b. The structure prediction section 102a fetches RNA sequences from the RNA sequence database 106a by a processing performed by the parsing section 102b (at a step SB-1), fits the selected grammar to the respective RNA sequences (at a step SB-2), and parses the RNA sequences (at a step SB-3).

The goodness-of-fit calculation section 102c calculates goodnesses of fit of derived parse trees. The structure prediction section 102a extracts, as RNA sequence candidates that could potentially form the secondary structures consistent with the structural topology modeled by the designated grammar, the RNA sequences from which the parse trees having the goodnesses of fit that satisfy preset conditions are derived (at a step SB-4).

The structure prediction section 102a outputs the extracted RNA sequences to the output device 114 through the input and output control interface 108 as RNA sequences that may possibly have the secondary structures consistent with the structural topology modeled by the grammar (at a step SB-5). The RNA sequences of similar structures extraction processing is thereby finished.

[Common Structure Extraction Processing]

The detail of a common structure extraction processing will be explained with reference to Fig. 9. Fig. 9 is a conceptual diagram which illustrates one example of a common structure extraction process performed by the system according to the embodiment.

The structure prediction section 102a fetches one or two or more RNA sequences from the RNA sequence database 106a (at steps SC-1 and SC-2), and applies one or two or more grammars, which are fetched from the grammar database 106b (at a step SC-3), to each RNA sequence (at a step SC-4). The RNA sequence analyzer 100 may perform either a parallel processing or a sequential processing for fetching the RNA sequences and the grammars and parsing.

The goodness-of-fit calculation section 102c calculates goodnesses of fit of derived parse trees, and extracts the RNA sequences, from which the parse trees having the goodness of fit that satisfy preset conditions are derived, by a processing performed by the common structure matrix creation section 102f (at a step SC-5).

The common structure matrix creation section 102f displays structural topologies modeled by the grammars and the RNA sequences in the two-dimensional matrix, gives marks to lattice parts corresponding to the extracted RNA sequences and structural topologies in the two-dimensional matrix, and thereby visualizes structural topologies common to the RNA sequences (at a step SC-6).

The marks may be given in a specific color to the target lattice parts as illustrated in Fig. 9, or by a specific symbol (e.g., ) or a letter (e.g., "Y") in the target lattice parts. By doing so, if continuous marks are present in a longitudinal direction (in a second structural topology column in the example illustrated in Fig. 9), for example, it is possible to visually recognize that these structural topologies are common to the RNA sequences. The common structure extraction processing is thereby finished.

[Structure Similarity Calculation Processing]

The detail of a structure similarity calculation processing will be explained with reference to Fig. 10. Fig. 10 is a conceptual diagram which illustrates one example of the structure similarity calculation process performed by the system according to the embodiment.

The user inputs multiple (two in the example illustrated in Fig. 10) RNA sequences, for which a similarity is to be calculated, to the RNA sequence analyzer 100 through the input device 112 (at a step SE-1).

The similarity calculation section 102d fetches one or two or more grammars from the grammar database 106b (at a step SE-2), and parses the input RNA sequences by applying the grammars to the respective input RNA sequences, by a processing performed by the parsing section 102b (at a step SE-3). The goodness-of-fit calculation section 102c calculates goodnesses of fit of derived parse trees (at a step SE-4).

The similarity calculation section 102d performs a vector operation, an inner product calculation, and the like for each RNA sequence while making the parse trees, derived by applying the grammars to the input RNA sequences, and the goodnesses of fit of the derived parse trees (if the goodnesses of fit are not derived, special values are set) correspond to one another (at a step SE-5), thereby calculating a similarity among the RNA sequences (at a step SE-6).

It is assumed, for example, that the input i RNA sequences are $RNA_1$, $RNA_2$, ..., and $RNA_i$, the N grammars stored in the grammar database 106b are $G_1$, $G_2$, ..., and $G_N$, and the goodness of fit obtained when the parsing of an RNA sequence x based on a grammar g is successful is r (x, g). It is also assumed herein that the goodness of fit is a real value and that as a goodness of fit is higher, the RNA sequence can form the structure having the goodness of fit more easily.

It is also assumed herein that for a goodness-of-fit vector $R_i$ with respect to an input RNA sequence $RNA_j$, a

$k^{th}$ element $R_j[k]$ of the vector $R_j$ is r ($RNA_j$, $G_k$) obtained when parsing $RNA_j$ based on $G_k$ is successful, or "X" obtained when the parsing fails.

The similarity calculation section 102d performs the similarity calculation processing by the following method. The user inputs goodness-of-fit vectors $R_1$ and $R_2$ with respect to two RNA sequences first.

The similarity calculation section 102d obtains similarity vectors $S_1$ and $S_2$ and the penalty P. The "penalty P" represents the number of elements k for which only one of elements $R_1[k]$ and $R_2[k]$ is indicated as "unsuccessful parsing (X)". The "similarity vectors $S_1$ and $S_2$" represent vectors obtained by extracting only elements for which neither $R_1[k]$ nor $R_2[k]$ are indicated as "unsuccessful parsing (X)". Fig. 12 is an illustration for explaining concepts of the penalty P and the similarity vectors $S_1$ and $S_2$.

The similarity calculation section 102d then calculates a distance D between the similarity vectors $S_1$ and $S_2$ by the following method. The number of elements (vector dimensions) of the similarity vectors $S_1$ and $S_2$ is assumed as M. Using the Euclidian distance generally used in similarity calculation, the distance D is calculated according to the following Equation.

$$D = sqrt(\Sigma\{(S_1[k] - S_2[k])^2\})$$

(where sqrt represents a square root and $\Sigma$ represents a sum for k = 1 to M)

If the distance D is large, the similarity is low. If the penalty P is large, the similarity is low. Therefore, using the penalty P and the distance D, the similarity Sim is calculated according to the following Equation.

$$Sim = a^p/D$$

(where a represents a constant (0<a<1))

Sim is output as the similarity. If the constant a is set low, more importance is attached to the penalty P than the distance D. The similarity calculation processing is thereby finished.

[Gene Prediction Processing]

The detail of a gene prediction processing will be explained with reference to Fig. 11. Fig. 11 is a conceptual diagram which illustrates one example of the gene prediction process performed by the system according to the embodiment.

The user first inputs a DNA sequence having an unknown gene part to the RNA sequence analyzer 100 through the input device 112. If so, the RNA sequence analyzer 100 automatically transforms the input DNA sequence to an RNA sequence transcribed from the DNA sequence (hereinafter, "predicted RNA sequence") and thereby produces the predicted RNA sequence by a processing performed by the gene prediction section 102g (at a step SF-1). The user may input the DNA sequence by selecting a desired DNA sequence from the external database in the external system 200 or the in-house database or by directly inputting the desired sequence.

If the structure prediction section 102a inputs this predicted RNA sequence to the parsing section 102b (at a step SF-2), one or two or more grammars are fetched from the grammar database 106b by a processing performed by the parsing section 102b (at a step SF-3), and the respective grammars are made fit to the predicted RNA sequence (at a step SF-4).

The goodness-of-fit calculation section 102c calculates goodnesses of fit of parse trees derived by the parsing section 102b (at a step SF-5). The gene prediction section 102g predicts a DNA sequence part corresponding to the predicted RNA sequence, from which the parse tree having the goodness of fit that satisfies preset conditions is derived, as a gene candidate (at a step SF-6). Namely, the part of the predicted RNA sequence among the DNA sequence is output as an area having a high probability of being a gene.

Thus, it is possible to predict that there is a probability that the part of the DNA sequence corresponding to the predicted RNA sequence having a known topologyshould be a gene. The gene prediction processing is thereby finished.

[Embodiment]

The embodiment of the present invention will be explained with reference to Figs. 13 to 23.

1 Preparation

In this section, some specific RNA secondary structure topologies are defined and grammars that model the respective topologies are specified in preparation for the embodiment. In this embodiment, for convenience of explanation, context free grammars are used. However, even if tree grammars for RNA (Literature 1) having a higher modeling capability are used, the following examples can be similarly applied.

1.1 Secondary Structure Topology

Two RNA secondary structure topologies illustrated in Fig. 13 will be considered.

A stem loop includes a stem (H(a)) and a hairpin loop (L(a)). A double-parallel-stem loop includes two stem loops arranged in parallel. The double-parallel-stem loop also includes a loop part (I(b)) that connects the two stems together, other than the stems ($H_1$(b) and $H_2$(b)) and the hairpin loops ($L_1$(b) or $L_2$(b)).

More specific features of these structural topologies can be considered. For example, it is possible to consider such topologies as those having more detailed features including size restrictions to each stem and each loop, whether a mismatching (an internal loop or a bulge loop) is permitted for the base pair constituting the stem, and whether a specific base sequence is present at a specific location. In this embodiment, therefore, RNA secondary structure topologies $T_1$ and $T_2$ having the following features will be defined.

Topology $T_1$

- A stem loop structure (see Fig. 13(a)) having the following features.
- The base pair constituting the stem (H(a)) does not include mismatches.
- The size of the stem (H(a)) is more than or equal to one base-pairs in length.
- The length of the hairpin loop (L(a)) is more than or equal to one base.

Topology $T_2$

- A double-parallel-stem loop structure (see Fig. 13(b)) having the following features.
- Two topologies $T_1$ are connected in parallel.
- The length of the loop (I(b)) between the stem ($H_1$(b)) and the stem ($H_2$(b)) is more than or equal to one base.

1.2 Secondary Structure Modeling by Context Free Grammars

The context free grammars modeling the two topologies $T_1$ and $T_2$ are defined below. Context Free Grammars are defined as a four tuple.

$$G = (N, \Sigma, P, S)$$

N represents a finite set of nonterminal symbols, $\Sigma$ represents a finite set of terminal symbols, P represents a finite set of production rules, and S represents a start symbol.

However, it is assumed in this embodiment that $\Sigma$ is {a, u, g, c}, the start symbol is S, and N includes only nonterminal symbols that appear in the production rules P. Therefore, by designating P only, the context free grammar G can be defined. For the sake of convenience, if the context free grammar G is specified, only the finite set of production rules P are designated herein.

(1) The topology $T_1$ is modeled by a context free grammar $G_1$ including the following production rules.

$$S \rightarrow xH\bar{x}$$

$$H \rightarrow xH\bar{x}|L$$

$$L \rightarrow xL \,|\, x$$

In the rules, $x \in \Sigma$, and $\bar{x}$ represents a complementary base of x which constitutes, together with x, a base pair.

Namely, if only a Watson-Crick base pair is assumed, the first production rule is equivalent to the following rule.

$$S \rightarrow aHu \,|\, uHa \,|\, gHc \,|\, cHg$$

If a non-Watson-Crick base pair is permitted, a rule of $S \rightarrow gHu$ or the like may be added.

In $G_1$, base pairs (constituting the stem) are produced based on the following rules:

$$S \rightarrow xH\bar{x} \text{ and } H \rightarrow xH\bar{x}.$$

In addition, bases (constituting the loop) that do not form the base pairs are produced based on the rules, $L \rightarrow xL$ and $L \rightarrow x$. Thus, the RNA secondary structures can be produced based on the grammar $G_1$. In this manner, for a context free grammar G, a set SS(G) of all RNA secondary structures that can be produced based on G could be specified.

The statement "$G_1$ models the topology $T_1$" holds if and only if "$G_1$ can produce all the RNA secondary structures consistent with the topology $T_1$, and all the RNA secondary structures that can be produced based on $G_1$ are consistent with topology $T_1$".

These are quite obvious from derivation processes in $G_1$. All the possible derivation in $G_1$ are in the following form.

In the following derivation, n and I satisfy n 1 and I 1.

$$S \rightarrow x_1 H \overline{x_1}$$

$$\rightarrow x_1 x_2 H \overline{x_2 x_1} \rightarrow K \rightarrow x_1 x_2 \, K \, x_n H \overline{x_n} \, K \, \overline{x_2 x_1}$$

$$\rightarrow x_1 x_2 \, K \, x_n L \overline{x_n} \, K \, \overline{x_2 x_1}$$

$$\rightarrow x_1 x_2 \, K \, x_n y_1 L \overline{x_n} \, K \, \overline{x_2 x_1}$$

$$\rightarrow K \rightarrow x_1 x_2 \, K \, x_n y_1 \, K \, y_{l\text{-}1} L \overline{x_n} \, K \, \overline{x_2 x_1}$$

$$\rightarrow x_1 x_2 \, K \, x_n y_1 \, K \, y_{l\text{-}1} y_1 \overline{x_n} \, K \, \overline{x_2 x_1}$$

In the derivation above, $x_1 x_2 ... x_n$ and $\bar{x}_n ... \bar{x}_2 \bar{x}_1$ correspond to the stem, and $y_1 ... y_{l\text{-}1} y_l$ corresponds to the hairpin loop. Since n and I satisfy n 1 and I 1, the size of the stem is more than or equal to one base-pairs in length and that of the hairpin loop is more than or equal to one base.

This demonstrates, therefore, that $G_1$ can model $T_1$.

(2) The topology $T_2$ is modeled by a context free grammar $G_2$ including the following production rules.

$$S \rightarrow S_1 I S_2$$

$$S_1 \rightarrow xH\bar{x}$$

$$S_2 \rightarrow xH\bar{x}$$

$$H \rightarrow xH\bar{x}|L$$

$$L \rightarrow xL|x$$

$$I \rightarrow xL$$

A context free grammar $G_0$ including the following production rules is a universal context free grammar that can produce all RNA secondary structures that can be produced based on context free grammars.

$$S \rightarrow SS|xS\bar{x}|xS|Sx|x|\lambda$$

In the rule, $\lambda$ represents a null character. For example, $G_0$ can simulate any derivation in $G_1$. Namely, the following derivation can be carried out by $G_0$.

$$S \rightarrow x_1 S \overline{x_1}$$

$$\rightarrow x_1 x_2 S \overline{x_2 x_1} \rightarrow K \rightarrow x_1 x_2 \ K \ x_n S \overline{x_n} K \ \overline{x_2 x_1}$$

$$\rightarrow x_1 x_2 \ K \ x_n S \overline{x_n} \ K \ \overline{x_2 x_1}$$

$$\rightarrow x_1 x_2 \ K \ x_n y_1 S \overline{x_n} \ K \ \overline{x_2 x_1}$$

$$\rightarrow K \rightarrow x_1 x_2 \ K \ x_n y_1 \ K \ y_{l-1} S \overline{x_n} \ K \ \overline{x_2 x_1}$$

$$\rightarrow x_1 x_2 \ K \ x_n y_1 \ K \ y_{l-1} y_1 \overline{x_n} \ K \ \overline{x_2 x_1}$$

In this derivation, the produced RNA secondary structures are entirely equal to those produced according to $G_1$ except for the nonterminal symbols. It is thus demonstrated that $G_0$ can produce all secondary structures that can be produced based on $G_1$. In other words, the following inclusive relation holds.

$$SS \ (G_0) \supseteq SS \ (G_1)$$

It is also obvious that the following relation holds for any context free grammar G.

$$SS \ (G_0) \supseteq SS \ (G)$$

It is assumed hereinafter that the overall secondary structures produced by such a universal grammar are "all secondary structures".

1.3 Parse Tree and Goodness of Fit

A question of whether a given RNA sequence can form a secondary structure that satisfies the properties of a given RNA secondary structural topology corresponds to a question of whether a grammar that models a target topology can derive a target sequence. This question can be solved by a parsing algorithm for the grammars.
The parsing algorithm determines whether a given grammar can derive a given sequence. If it is determined that the given grammar can derive the given sequence, derivation processes of the derivation, that is, a parse tree is output. In the framework of the grammar that models the secondary structures, the parse tree corresponds to the specific secondary structure. Therefore, it can be interpreted that the parsing algorithm outputs a specific secondary structure consistent with the target topology if exists.

It is now considered whether an RNA sequence $s_1$ = ggggaaacccc can form the secondary structures consistent with the topologies $T_1$ and $T_2$.

The sequence $s_1$ can be derived by $G_1$ as follows. This shows that the sequence $S_1$ can form the secondary structure consistent with $T_1$.

$$S \rightarrow gHc \rightarrow ggHcc \rightarrow gggHccc \rightarrow ggggHcccc \rightarrow ggggLcccc \rightarrow$$

$$ggggaLcccc \rightarrow ggggaaLcccc \rightarrow ggggaaacccc \qquad \cdots (1)$$

Alternatively, the sequence $s_1$ can be derived by $G_1$ as follows.

$$S \rightarrow gHc \rightarrow ggHcc \rightarrow gggHccc \rightarrow gggLccc \rightarrow ggggLccc \rightarrow ggggaLccc \rightarrow$$

$$ggggaaLccc \rightarrow ggggaaaLccc \rightarrow ggggaaacccc \qquad \cdots (2)$$

It is noted, however, that the sequence $s_1$ cannot be derived by $G_2$. This follows that the sequence $s_1$ cannot form the secondary structure consistent with the topology $T_2$.

Fig. 14 illustrates parse trees corresponding to the two derivations above, deriving $s_1$ based on $G_1$, and secondary structures corresponding to the respective parse trees. That is, if the sequence $s_1$ is derived by the derivation process (1), the parse tree and the secondary structure illustrated in Fig. 14(1) are produced. If the sequence $s_1$ is derived by the derivation process (2), the parse tree and the secondary structure illustrated in Fig. 14(2) are produced.

If more than one parse trees are obtained as shown in the example of Fig. 14, it is necessary to determine which parse tree, i.e., which secondary structure is to be output as a result. Accordingly, it is necessary to allocate scores to the respective parse trees (or secondary structures) based on a certain evaluation function, and to rank the parse trees (or secondary structures) according to the score. As such scores, different evaluation functions may be employed according to grammars or an absolute evaluation function that does not depend on grammars may be employed. The scores will be referred to as "goodnesses of fit" hereinafter.

Examples of goodness-of-fit evaluation methods used so far will be shown below. However, the goodnesses of fit used by the present invention are not limited to the examples.

(1) Goodness-of-Fit Evaluation based on Base-pairs

Generally, an RNA molecule is stabilized in terms of energy thanks to a hydrogen bond generated when forming a base pair. Therefore, in this evaluation method, the secondary structure having more base pairs is simply given a higher priority. That is, as the goodness of fit of a parse tree, the number of base-pairs of the corresponding secondary structure is used. According to this evaluation method, if the goodnesses of fit of the parse trees in the above examples are evaluated, the goodness of fit of the parse tree illustrated in Fig. 14(1) is 3, and that of the parse tree illustrated in Fig. 14(2) is 2. Therefore, the structure illustrated in (1), and having a higher goodness of fit is adopted.

As a classical algorithm based on this evaluation method, there is known Nussinov's folding algorithm explained in Nussinov. R., Piecxenk, G., geiggs, j. R., and Kleitman, D.J.: "Algorithms for loop matchings" in *SIAM journal of Applied Mathematics*, 35, pp. 68-82, 1978.

(2) Goodness-of-Fit Evaluation based on Free Energy increments ($\Delta G$)

In order to evaluate a physico-chemical stability of an RNA secondary structure, there is known a calculation method using free energy ($\Delta G$) parameters determined by a thermodynamic experiment conducted to a small model RNA molecule. The free energy increments ($\Delta G$) of a certain secondary structure is approximated to a sum of free energies of secondary structure elements, such as base pairs and loops, which constitute the secondary structure. According to the free energy parameters, the structure is made stable by the base pairs and made unstable by the loops. Detailed parameters for the respective secondary structure elements are shown in Turner, D.H., Sugimoto, N., Jaeger, J.A., Longfellow, C.E., Freier, S.M., and Kierzek, R.: "Improved parameters for prediction of RNA structure" in *Cold Spring Harbor Symposia Quantitative Biology,* 52, pp. 123-133, 1987. In this specification, Fig. 15 illustrates free energies of base pairs, and Fig. 16 illustrates free energies of loops.

Using the free energy parameters, free energy increments ($\Delta G$) of the structures (1) and (2) illustrated in Fig. 14 are calculated as follows.

$$\Delta G \text{ (structure (1))} = \Delta G \text{ (gc, gc)} + \Delta G \text{ (gc, gc)}$$

$$+ \Delta G \text{ (gc, gc)}$$

$$+ (\Delta G) \text{ (size 3 hairpin loop)}$$

$$= (-2.9) + (-2.9) + (-2.9)$$

$$+ 7.4 = -1.3$$

$$\Delta G \text{ (structure (2))} = \Delta G \text{ (gc, gc)} + \Delta G \text{ (gc, gc)}$$

$$+ \Delta G \text{ (size 5 hairpin loop)}$$

$$= (-2.9) + (-2.9) + 4.4 = -1.4$$

Care should be taken here to the method of calculating the free energies of base pairs. One energy value is allocated to two base pairs that are continuously stacked. Namely, for the calculation of the free energy of the structure (1), $\Delta G$(gc, gc) is calculated for the first and the second gc base pairs from 5' side, $\Delta G$(gc, gc) is calculated for the second and the third gc base pairs, and $\Delta G$(gc, gc) is calculated for the third and the fourth gc base pairs. For the calculation of the free energy of the structure (2), by contrast, $\Delta G$(gc, gc) is calculated for the first and the second gc base pairs from 5' side, and $\Delta G$(gc, gc) is calculated for the second and the third gc base pairs.

If it is specified that the goodness of fit of a parse tree is $-\Delta G$, then the goodness of fit of the structure (1) is 1.3 and that of the structure (2) is 1.4. As a result, the structure (2) having a higher goodness of fit is adopted.

As a typical RNA secondary structure prediction system based on $\Delta G$, there is known Zuker's Mfold (Literature 3).

(3) Goodness-of-Fit Evaluation based on Derivation Probability

Stochastic Grammars are formal grammars having production rules to which application probabilities are added, respectively. Suppose a stochastic context free grammar $G_1$ having the production rules of the grammar $G_1$ to which the following probabilities p are added will be considered.

$$p \text{ (S} \rightarrow \text{aHu)} = 0.2$$

$$p \text{ (S} \rightarrow \text{uHa)} = 0.2$$

$$p \text{ (S} \rightarrow \text{gHc)} = 0.3$$

$$p \text{ (S} \rightarrow \text{cHg)} = 0.3$$

$$p \text{ (H} \rightarrow \text{aHu)} = 0.2$$

$$p\ (H \rightarrow uHa) = 0.2$$

$$p\ (H \rightarrow gHc) = 0.3$$

$$p\ (H \rightarrow cHg) = 0.2$$

$$p\ (H \rightarrow L) = 0.1$$

$$p\ (L \rightarrow aL) = 0.2$$

$$p(L \rightarrow uL) = 0.2$$

$$p(L \rightarrow gL) = 0.15$$

$$p(L \rightarrow cL) = 0.15$$

$$p(L \rightarrow a) = 0.1$$

$$p(L \rightarrow u) = 0.1$$

$$p\ (L \rightarrow g) = 0.05$$

$$p(L \rightarrow c) = 0.05$$

A derivation probability of the sequence $s_1$ by this $G_1$ is calculated as follows. Namely, the derivation probability of the sequence $s_1$ having the structure (1) is calculated as follows.

$$p\ (S \rightarrow gHc) \times p\ (H \rightarrow gHc) \times p\ (H \rightarrow gHc) \times p\ (H \rightarrow gHc) \times p\ (H \rightarrow L) \times p\ (L$$

$$\rightarrow aL) \times p\ (L \rightarrow aL) \times p\ (L \rightarrow a) = 0.3 \times 0.3 \times 0.3 \times 0.3 \times 0.1 \times 0.2 \times 0.2 \times 0.1$$

$$= 0.00000324$$

The derivation probability of the sequence $s_1$ having the structure (2) is calculated as follows.

$$p\ (S \rightarrow gHc) \times p\ (H \rightarrow gHc) \times p\ (H \rightarrow gHc) \times p\ (H \rightarrow L) \times p\ (L \rightarrow gL) \times$$

$$p\ (L \rightarrow aL) \times p\ (L \rightarrow aL) \times p\ (L \rightarrow aL) \times p\ (L \rightarrow C) = 0.3 \times 0.3 \times 0.3 \times 0.1 \times$$

$$0.15 \times 0.2 \times 0.2 \times 0.2 \times 0.05 = 0.000000162$$

Accordingly, if a natural logarithm of a derivation probability is used as the goodness of fit of a parse tree, then the goodness of fit of the parse tree (1) is 1n0.00000324 = -12.6, that of the parse tree (2) is 1n0.000000162 = -15.6. As a result, the structure (1) having a higher goodness of fit is adopted.

Probabilistic parameters to be added to the respective production rules, based on which this evaluation method is executed, may be learned by a maximum likelihood estimation method, an inside-outside algorithm or the like, or may be estimated by heuristics or the like. In a Literature of Sakakibara et al.: "Stochastic Context-fee Grammars for tRNA modeling" in *Nucleic Acids Research,* 22, 5112-5120, 1994, for example, a method for learning stochastic context free grammars that models tRNA structures from a plurality of tRNA sequences is developed.

While several goodness-of-fit evaluation methods have been explained above, $-\Delta G$ is used as the goodness of fit hereinafter.

Next, It could be considered whether the RNA sequence $s_2$= gcccauaggcaaagccuaugggc can form secondary structures consistent with the topologies $T_1$ and $T_2$. Similarly to the above, it may be determined whether $s_2$ can be derived by $G_1$ and $G_2$. As a conclusion, the sequence $s_2$ can be derived by either $G_1$ or $G_2$. Further, multiple derivations exist for each grammar. Fig. 14 illustrates optimum parse trees and corresponding secondary structures with $-\Delta G$ used as the goodness-of-fit index for the grammars $G_1$ and $G_2$, respectively.

Free energy increments ($\Delta G$) for the respective structures are calculated as follows.

$$\Delta G \text{ (structure (1))} = \Delta G \text{ (gc, cg)} \times 2 + \Delta G \text{ (cg, cg)}$$

$$\times 2 + \Delta G \text{ (cg, au)} + \Delta G \text{ (au, ua)}$$

$$+ \Delta G \text{ (ua, au)} + \Delta G \text{ (au, gc)}$$

$$+ \Delta G \text{ (gc, gc)} + \Delta G \text{ (size 3 hairpin loop)}$$

$$= (-3.4) \times 2 + (-2.9) \times 2 + (-1.8)$$

$$+ (-0.9) + (-1.1) + (-1.7)$$

$$+ (-2.9) + 7.4 = -13.6$$

$$\Delta G \text{ (structure (2))} = \Delta G \text{ (gc, cg)} \times 2 + \Delta G \text{ (cg, cg)} \times 2$$

$$+ \Delta G \text{ (size 4 hairpin loop)} \times 2$$

$$= (-3.4) \times 2 + (-2.9) \times 2 + 5.9 \times 2$$

$$= -6.7$$

It is thus demonstrated that the goodness of fit of the parse tree having the optimum RNA secondary structure that may possibly be formed by the sequence $s_2$ among those consistent with the topology $T_1$ is 13.6. In addition, the goodness of fit of the parse tree having the optimum RNA secondary structure that may possibly be formed by the sequence $s_2$ among those consistent with the topology $T_2$ is 6.7. If the sequence $s_2$ is parsed using the universal grammar $G_0$, the structure (1) is found as the optimum structure. This follows that the structure (1) is the optimum structure among "all secondary structures". By thus parsing based on the universal grammar, it is possible to find the optimum structure among all secondary structures.

"A parsing unit that applies an RNA sequence to a formal grammar and derives a parse tree, a goodness-of-fit calculation unit that calculates a goodness of fit of the parse tree derived by the parsing unit, and an optimum secondary structure output unit that outputs a secondary structure corresponding to the parse tree having the optimum goodness of fit", which units form the basis for the present invention, are generally implemented by a parsing algorithm having goodness-of-fit calculation incorporated therein. This parsing algorithm will be referred to as "structure prediction algorithm". The structure prediction algorithm based on the RNA tree grammar with G used as a goodness-of-fit index is disclosed in Literature 1.

2. Embodiment of the Present Invention

In this section, an embodiment in which the RNA sequences $s_1$ and $s_2$, the topologies $T_1$ and $T_2$, the context free grammars $G_0$, $G_1$, and $G_2$ for modeling the topologies, and - G serving as the goodness of fit are used will be explained.

First of all, "the grammar storage unit that stores structural topologies of RNA secondary structures and production grammars corresponding to the respective structural topologies" store a name of a structural topology such as (Leu-tRNA, G') or (16S rNA, G") associated with a grammar that models the given structural topology. In this embodiment, the unit is assumed as a grammar DB which may include (stem loop $T_1$, $G_1$) and (double-parallel-stem loop $T_2$, $G_2$). In addition, it is assumed that an RNA sequence DB including the RNA sequences $s_1$ and $s_2$ is used.

(1) Output of structure candidates by a grammar and goodness-of-fit calculation

If the user is to grasp structural topologies that may possibly be formed by a given RNA sequence in a descending order of goodness of fit, the user can do so in the following procedures according to the present invention. In this example, an instance in which the input sequence is $s_2$ and the target topology sets are $T_1$ and $T_2$ will be shown.

Procedure 1) An RNA sequence is designated from the sequence DB or directly input. In this example, the sequence $s_2$ is designated.
Procedure 2) Target topology sets (grammar sets) are selected from the grammar DB. In this example, $T_1$ and $T_2$ are selected.
Procedure 3) A threshold for the goodness of fit is set. The threshold may be set for each topology (grammar) obtained in the procedure 2 or one common threshold may be set. In this example, 10 is set for $T_1(G_1)$ and 5 is set for $T_2(G_2)$.
Procedure 4) The sequence obtained in the procedure 1 is parsed based on each grammar obtained in the procedure 2, and the parse tree having the maximum goodness of fit is obtained for the sequence. In this example, $s_2$ is parsed based on $G_1$, and the parse tree having the maximum goodness of fit 13.6 is obtained (see Fig. 17(1)).
Further, $s_2$ is parsed based on $G_2$, and the parse tree having the maximum goodness of fit of 6.7 is obtained (see Fig. 17(2)).
Procedure 5) Among the parse trees obtained in the procedure 4, those having the goodnesses of fit equal to or higher than the thresholds obtained in the procedure 3 are sorted in the descending order of goodness of fit. The goodness of fit 13.6, obtained based on $G_1$ in the procedure 4, of the parse tree 1 is higher than the threshold 10 set for $G_1$ in the procedure 3. Therefore, the parse tree 1 is to be sorted. The goodness of fit 6.7, obtained based on $G_2$ in the procedure 4, of the parse tree 2 is higher than the threshold 5 set for $G_2$ in the procedure 3. Therefore, the parse tree 2 is to be sorted. By thus sorting the sort target parse trees in the descending order of goodness of fit, the parse tree 1 ranks ahead the parse tree 2.
Procedure 6) Names, goodnesses of fit, parse trees (secondary structures), and the like of corresponding topologies are output in a descending order of parse trees sorted in the procedure 5. The stem loop $T_1$, the goodness of fit 13.6, and the secondary structure illustrated in Fig. 17(1) are output so as to correspond to the parse tree 1. The double-parallel-stem loop $T_2$, the goodness of fit 6.7, and the secondary structure illustrated in Fig. 17(2) are output so as to correspond to the parse tree 2.

As a result, structure candidates fit to $s_2$ in the selected topology sets are output as illustrated in Fig. 18.
According to the conventional secondary structure prediction program, optimum or optimal secondary structures among those that may possibly be formed by the given sequence are sequentially output. Therefore, the user is required to determine what topologies the output structures have. According to the present invention, the structures and the topologies can be output. It is, therefore, expected to greatly reduce labor required to see the prediction result.
Further, if the present invention is carried out, the procedures for the check are not necessarily, strictly equal to those explained above. For example, the order of the procedures 1 and 2 may be changed, or the selection of the parse trees based on the thresholds in the procedure 5 may be included in the parsing in the procedure 4.

(2) Output of sequence candidates of similar structures.

If the user is to search for RNA sequences that may possibly have secondary structures consistent with a given structural topology, the user can do so in the following procedures according to the present invention. In this example, an instance in which the input topology is $T_2$ and the target sequence sets are $s_1$ and $s_2$ will be shown.

Procedure 1) A topology (grammar) is selected from the grammar DB. In this example, $T_2$ ($G_2$) is selected.
Procedure 2) A threshold for the goodness of fit is set. In this example, 5 is selected as the threshold.
Procedure 3) Target RNA sequence sets are selected from the sequence DB or directly input. In this example, $s_1$ and $s_2$ are selected.
Procedure 4) Each sequence obtained in the procedure 3 is parsed based on the grammar obtained in the procedure 1, and the parse tree having the optimum goodness of fit is obtained for the sequence. In this example, $s_1$ is

parsed based on $G_2$, but the parse tree cannot be derived from $s_1$. $s_2$ is parsed based on $G_2$, and the parse tree having the optimum goodness of fit 6.7 is obtained (see Fig. 17(2)).

Procedure 5) Among the parse trees obtained in the procedure 4, sequences corresponding to those having the goodnesses of fit equal to or higher than the threshold obtained in the procedure 4 are output. The parse tree derived from $s_2$ having the goodness of fit 6.7 derived based on $G_2$ and obtained in the procedure 4 is higher than the threshold 5 set in the procedure 2. Therefore, $s_2$ is output. As a result, a sequence candidate that may possibly have the selected topology is output as illustrated in Fig. 19.

If the present invention is carried out, the procedures for the search are not necessarily, strictly equal to those explained above. For example, the order of procedures 1, 2, and 3 may be arbitrarily exchanged, or the procedure 5 may be included in the parsing in the procedure 4.

(3) Extraction of common structure

If the user is to search for structural topologies common to a certain RNA sequence set, the user can do so in the following procedures according to the present invention. In this example, an instance in which the input sequence sets are $s_1$ and $s_2$ and the target topology sets are $T_1$ and $T_2$ will be shown.

Procedure 1) RNA sequence sets are designated from the sequence DB or directly input. In this example, $s_1$ and $s_2$ are designated.

Procedure 2) Target topology sets (grammar sets) are selected from the grammar DB. In this example, $T_1$ ($G_1$) and $T_2$ ($G_2$) are selected.

Procedure 3) A threshold for the goodness of fit is set. The threshold may be set for each topology (grammar) obtained in the procedure 2 or one common threshold may be set. In this example, a common threshold 0 is set.

Procedure 4) Each sequence obtained in the procedure 1 is parsed based on each grammar obtained in the procedure 2, and the parse tree having the optimum goodness of fit is obtained for the sequence.

In this example, $s_1$ is parsed based on $G_1$, and the parse tree having the optimum goodness of fit 1.4 is obtained (see Fig. 14(2)).

$s_1$ is parsed based on $G_2$, but the parse tree cannot be derived from $s_1$.

$s_2$ is parsed based on $G_1$, and the parse tree having the optimum goodness of fit 13.6 is obtained (see Fig. 17(1)).

$s_2$ is parsed based on $G_2$, and the parse tree having the optimum goodness of fit 6.7 is obtained (see Fig. 17(1)).

Procedure 5) Among the parse trees obtained in the procedure 4, those having the goodnesses of fit equal to or higher than the threshold are extracted. All the parse trees obtained in the procedure 4 have the goodnesses of fit higher than the threshold 0 obtained in the procedure 3. Therefore, all the parse trees obtained in the procedure 4 are extracted.

Procedure 6) A matrix in which the sequence sets obtained in the procedure 1 are arranged in rows and the topology sets obtained in the procedure 2 are arranged in columns, and which includes the goodnesses of fit of the parse trees obtained in the procedure 5 as elements, is created. Thus, the matrix illustrated in Fig. 20 is obtained.

If the matrix obtained based on the results is output, it is possible to easily check the structural topologies common to the target sequence sets. Alternatively, if the following additional procedures are executed, the common structure candidates can be output in an order.

Procedure 7) A score is calculated for each column of the matrix obtained in the procedure 6, i.e., each topology. For example, if the number of effective row elements is calculated for each column and the calculation result is set as a score, then the score of $T_1$ is 2 and that of $T_2$ is 1. If a sum of goodnesses of fit in rows is calculated for each column and set as a score, then the score of $T_1$ is 15.0 and that of $T_2$ is 6.7.

Procedure 8) The topologies are sorted and output in a descending order of scores obtained in the procedure 7. Whichever scores are adopted, the topologies are output in the order of $T_1$ and $T_2$.

Further, if the present invention is carried out, the procedures for the search are not necessarily, strictly equal to those explained above. For example, the order of the procedures 1 and 2 may be changed, or the procedure 5 may be included in the parsing in the procedure 4.

(4) Gene Finder

A sequence corresponding to an RNA gene tends to have quite a stable structure, so that the goodness of fit thereof is high. Therefore, according to the present invention, parsing is performed using the universal grammar, sequences having high goodnesses of fit are selected from the sequence DB and output as gene candidates. In this example, an instance in which the sequence sets are $s_1$ and $s_2$ will be shown.

Procedure 1) Target RNA sequence sets are designated from the sequence DB or directly input. In this example, $s_1$ and $s_2$ are designated.

Procedure 2) A threshold for the goodness of fit is set. In this example, 10 is set as the threshold.

Procedure 3) Each sequence obtained in the procedure 1 is parsed based on the universal grammar $G_0$, and the parse tree having the optimum goodness of fit is obtained.

$s_{1\,1}$ is parsed based on $G_0$, and the parse tree having the optimum goodness of fit 1.4 is obtained.

$s_2$ is parsed based on $G_0$, and the parse tree having the optimum goodness of fit 13.6 is obtained.

Procedure 4) Among the parse trees obtained in the procedure 3, the sequences corresponding to those having the goodnesses of fit equal to or higher than the threshold are output as gene candidates. Since the parse tree derived from $s_1$ and obtained in the procedure 3 is lower than the threshold 10, $s_1$ is not output. Since the parse tree derived from $s_2$ and obtained in the procedure 3 is greater than the threshold 10, $s_2$ is output as a gene candidate.

If the present invention is carried out, the procedures for the gene finding are not necessarily, strictly equal to those explained above. For example, the order of the procedures 1 and 2 may be changed, or the procedure 4 may be included in the parsing in the procedure 3.

(5) Output of RNA sequences potentially having the similar structures as that of the given RNA sequence set

If the user is to search for RNA sequences that may possibly have the similar topology as that of a certain RNA sequence set, the user can do so according to the present invention in a combination of the invention (3) and the invention (2). In this example, an instance in which the input sequence is s = gcccaaaagggcagcccaaagggc, the target topology sets are $T_1$ and $T_2$, and the target sequence sets are $s_1$ and $s_2$ will be shown.

Procedure 1) An RNA sequence set is input. In this example, the sequence set including only s is input.

Procedure 2) Target RNA sequence sets are designated from the sequence DB. In this example, $s_1$ and $s_2$ are designated.

Procedure 3) Target topology sets (grammar sets) are selected from the grammar DB. In this example, $T_1$ ($G_1$) and $T_2$ ($G_2$) are selected.

Procedure 4) A threshold for the goodness of fit is set. The threshold may be set for each topology (grammar) obtained in the procedure 3 or a common threshold may be set. In this example, a common threshold 5 is set.

Procedure 5) Each RNA sequence obtained in the procedure 1 is parsed based on each grammar obtained in the procedure 2, and the parse tree having the optimum goodness of fit is obtained for the sequence. In this example, s is parsed based on $G_1$, and the parse tree having the optimum goodness of fit 3.1 is obtained. Fig. 21(1) illustrates a secondary structure corresponding to this parse tree. Further, s is parsed based on $G_2$, and the parse tree having the optimum goodness of fit 5.1 is obtained. Fig. 21(2) illustrates a secondary structure corresponding to this parse tree.

Procedure 6) Among the parse trees obtained in the procedure 5, the parse trees corresponding to those having the goodnesses of fit equal to or higher than the threshold obtained in the procedure 4 are extracted. Among the parse trees obtained in the procedure 5, the parse tree having the goodness of fit 5.1 obtained by parsing the RNA sequence based on $G_2$ is higher than the threshold 5. Therefore, this parse tree is extracted.

Procedure 7) A matrix in which the sequence sets obtained in the procedure 1 are arranged in rows and the topology sets obtained in the procedure 3 are arranged in columns, and which includes the goodnesses of fit of the parse trees obtained in the procedure 6 as elements, is created. Thus, the matrix illustrated in Fig. 22 is obtained.

Procedure 8) A score is calculated for each column of the matrix obtained in the procedure 6, i.e., each topology, and topologies are sorted in a descending order of scores. In this example, a sum of rows is calculated for each column and set as a score. However, the matrix includes only one row, so that the score of $T_1$ is undefined and that of $T_2$ is 5.1. If only the topologies having scores are sorted, only $T_2$ is obtained.

Procedure 9) Each sequence obtained in the procedure 2 is parsed based on the corresponding grammar in the order of topologies obtained in the procedure 8, and the parse tree having the optimum goodness of fit is obtained for each sequence. In this example, $s_1$ is parsed based on $G_2$, and the parse tree cannot be derived from $s_1$.

Further, $s_2$ is parsed based on $G_2$, and the parse tree having the optimum goodness of fit 6.7 is obtained (see Fig. 17(2)).

Procedure 10) Among the parse trees obtained in the procedure 9, sequences corresponding to those having goodnesses of fit equal to or higher than the threshold obtained in the procedure 4 are output. At this time, topologies and the scores for the topologies obtained in the procedure 8 are also output. The goodness of fit 6.7 of the parse tree derived from $s_2$ based on $G_2$ in the procedure 9 is higher than the threshold 5 obtained in the procedure 4. Therefore, $s_2$ is output, and $T_2$ and the score of $T_2$ of 5.1 are output.

The output illustrated in Fig. 23 is obtained by the above result.

Accordingly, it is seen that $s_2$ may possibly have a common structure to s for the topology $T_2$.

If the present invention is carried out, the procedures for the search are not necessarily, strictly equal to those explained above. For example, the procedures 1, 2, and 3 may be arbitrarily exchanged, the procedure 6 may be included in the parsing in the procedure 5, or the selection of the parse trees based on the threshold in the procedure 10 may be included in the parsing in the procedure 9.

[Other Embodiments]

The embodiment of the present invention has been explained so far. However, the present invention is not limited to the embodiment but may be carried out by various other embodiments within the scope of the technical concept set forth in the claims.

For example, the instance in which the RNA sequence analyzer 100 conducts the RNA sequence analysis method in a standalone fashion has been explained. Alternatively, the RNA sequence analyzer 100 may conduct the RNA sequence analysis method in response to a request from a client terminal constituted separately from the RNA sequence analyzer 100, and may return the processing result to the client terminal.

Further, the structure prediction section 102a may derive a parse tree by the parsing section 102 while the goodness-of-fit calculation section 102c is allowed to conduct the goodness-of-fit calculation. Namely, the parsing section 102b that derives the parse tree and the goodness-of-fit calculation section 102c that calculates the goodness of fit of the derived parse tree may be realized by one algorithm. By so constituting, numerous parse trees (in an exponential order relative to a sequence length) that may possibly be derived for RNA sequences and tree grammars are present. It is, therefore, possible to solve the disadvantage in that if the parse trees are derived, the goodnesses of fit of the parse trees are calculated, and then the parse trees are sorted, a calculation time and a storage capacity in the exponential order are required.

Further, among the respective processings explained in the embodiment, all of or part of the processings explained to be performed automatically may be performed manually or all of or part of the processings explained to be performed manually may be performed automatically by a well-known method.

The structure prediction section 102a, in particular, may be realized as a plurality of tasks and the respective tasks may perform parallel processings.

Furthermore, the processing procedures, control procedures, specific names, information including various pieces of registered data and parameters for search conditions and the like, screen examples, and database configurations explained above or illustrated in the drawings may be arbitrarily changed unless specified otherwise.

The respective constituent elements of the RNA sequence analyzer 100 illustrated in the drawings are functionally conceptual, and the RNA sequence analyzer 100 is not always required to be physically constituted as illustrated in the drawings.

For instance, all of or arbitrary part of the processing functions of the respective servers of the RNA sequence analyzer 100, particularly the respective processing functions performed by the control section can be realized by the CPU (Central Processing Unit) and programs interpreted and executed by the CPU, or can be realized as hardware based on wired logic. The programs are recorded on a recording medium to be explained later, and mechanically read by the RNA sequence analyzer 100 as needed.

The various databases and the like (the RNA sequence database 106a to the common structure matrix 106c) stored in the storage section 106a are storage units such as memory devices, e.g., a RAM and a ROM, fixed disk devices, e.g., a hard disk, a flexible disk, and an optical disk. They store various programs, tables, files, databases, webpage files, and the like used for various processings and provision of websites.

In addition, the RNA sequence analyzer 100 may be realized by connecting peripherals such as a printer, a monitor, and an image scanner to an information processing apparatus such as information processing terminals, e. g., well-known personal computers or workstations, and by installing software (including a program, data, or the like) for realizing the method of the present invention into the information processing apparatus.

The specific form of distribution and integration of the RNA sequence analyzer 100 is not limited to that illustrated in the drawings. All of or part of the RNA sequence analyzer 100 can be functionally or physically distributed or integrated in arbitrary units according to various loads and the like. For example, each database may be constituted independently as an independent database device, and part of the processings may be realized using a CGI (Common Gateway Interface).

Further, the program according to the present invention can be stored in a computer readable recording medium. It is assumed herein that examples of this "recording medium" include arbitrary "portable physical mediums" such as a flexible disk, a magneto-optical disk, a ROM, an EPROM, an EEPROM, a CD-ROM, an MO, and a DVD, arbitrary "fixed physical mediums" such as a ROM, a RAM, and an HD included in various computer systems, and "communication mediums" that temporarily hold the program such as a communication line or a carrier wave used when the program is transmitted through the network represented by a LAN, a WAN, or the internet.

The "program" is a data processing method described in an arbitrary language or by an arbitrary description method, and the form of the "program" is not limited but may be a source code, a binary code, or the like. The "program" is not limited to a program constituted as a single program. Examples of the "program" include a program constituted to be distributed as a plurality of modules or libraries, and a program that fulfils its function in cooperation with another program represented by the OS (Operating System). The specific configurations, reading procedures, install procedures after reading, and the like of the respective devices shown in the embodiment for reading the recording medium may be well-known configurations and procedures.

Furthermore, the network 300 functions to connect the RNA sequence analyzer 100 and the external system 200 to each other, and may include any one of, for example, the Internet, the Intranet, a LAN (which may be either wired or wireless), a VAN, a personal computer communication network, a public telephone network (which may be either analog or digital), a dedicated line network (which may be either analog or digital), a CATV network, a portable line exchange network/portable packet exchange network such as an IMT 2000 network, a GSM network, or a PDC/ PDC-P network, a wireless call network, a local wireless network such as Bluetooth, and satellite communications network such as CD, BS, or ISDB. That is, the present system can transmit and receive various pieces of data through an arbitrary network whether the system is wired or wireless.

As explained so far in detail, according to the present invention, structural topologies of RNA secondary structures with grammars corresponding to the structural topologies are stored, parse trees are derived by applying an RNA sequence to the grammars, goodnesses of fit of the derived parse trees are calculated, the parse trees having the goodnesses of fit that satisfy preset conditions are sorted in a descending order of the goodnesses of fit, and the sorted parse trees are output as secondary structure candidates of the RNA sequence. Thus, one sequence can be parsed based on multiple grammars. That is, the sequence is subjected to parsing and goodness-of-fit calculation for each parse tree derived from each grammar, thereby obtaining the goodness of fit for each structural topology. As a result, the grammars can be ranked by sorting the goodnesses of fit. Accordingly, the structural topologies for the grammars can be ranked. Hence, it is possible to provide the RNA sequence analyzer, the RNA sequence analysis method, the program, and the recording medium capable of ranking the structural topologies in a descending order of possibility for the given RNA sequence.

According to the present invention, a structural topology of RNA secondary structures with a grammar corresponding to the structural topology are stored, parse trees are derived by applying RNA sequences to the grammar, goodnesses of fit of the derived parse trees are calculated, and the RNA sequences, from which the parse trees having the goodness of fit that satisfy preset conditions are derived, are output as RNA sequence candidates that could potentially form the secondary structures consistent with the structural topology. Therefore, multiple sequences can be parsed based on one grammar. That is, for a given specific structural topology, a corresponding grammar is obtained. Using the grammar, all of or part of the RNA sequences stored in the RNA sequence database are parsed, respectively, and a group of the RNA sequences which can be successfully parsed with goodnesses of fit that satisfy preset conditions are output as a result. Hence, it is possible to provide the RNA sequence analyzer, the RNA sequence analysis method, the program, and the recording medium capable of searching for the RNA sequences that may possibly have the given specific structural topology.

According to the present invention, structural topologies of RNA secondary structures with grammars corresponding to the respective structural topologies are stored, parse trees are derived by applying RNA sequences to the grammars, goodnesses of fit of the derived parse trees, the RNA sequences from which the parse trees having the goodness of fit that satisfy preset conditions are derived are extracted, the structural topologies and the RNA sequences are displayed in a two-dimensional matrix, marks are given to lattice parts corresponding to the extracted RNA sequences and the structural topologies in the two-dimensional matrix, and the structural topologies common to the RNA sequences are thereby visualized. Hence, it is possible to provide the RNA sequence analyzer, the RNA sequence analysis method, the program, and the recording medium capable of easily finding the structures common to the RNA sequences.

According to the present invention, a structural topology of RNA secondary structures with a grammar corresponding to the structural topology are stored, RNA sequences transcribed from a DNA sequence input by a user are produced, parse trees are derived by applying the grammar to the produced RNA sequences, goodnesses of fit of the derived parse trees are calculated, and parts of the DNA sequence corresponding to the RNA sequences, from which the parse trees having the goodnesses of fit that satisfy preset conditions are derived, are predicted as gene candidates. Hence, it is possible to provide the RNA sequence analyzer, the RNA sequence analysis method, the program, and the recording medium capable of predicting that there is a probability that the part of the DNA sequence corresponding to the RNA sequence having known topology should be a gene part.

According to the present invention, a structural topology of RNA secondary structures with a grammar corresponding to the structural topology are stored, parse trees are derived by applying the grammar to RNA sequences, goodnesses of fit of the derived parse trees are calculated, a similarity among the RNA sequences is calculated based on the calculated goodnesses of fit. Hence, it is possible to provide the RNA sequence analyzer, the RNA sequence analysis method, the program, and the recording medium capable of easily obtaining the similarity of the RNA sequences based on the underlying RNA structures.

According to the present invention, structural topologies of RNA secondary structures with grammars corresponding to the structural topologies are stored, parse trees are derived by applying RNA sequences to the grammars, goodnesses of fit of the derived parse trees are calculated, the RNA sequences from which the parse trees having the goodnesses of fit that satisfy preset conditions are derived are extracted, a goodness-of-fit matrix which displays the structural topologies and the RNA sequences in a two-dimensional matrix, and which displays the goodnesses of fit on lattice parts corresponding to the extracted RNA sequences and the structural topologies in the two-dimensional matrix, is created, the structural topologies are sorted according to the goodnesses of fit for the goodness-of-fit matrix, other RNA sequences are parsed based on the grammar corresponding to an order of the sorted structural topologies, the parse trees having optimum goodnesses of fit are obtained, and the other RNA sequences corresponding to the parse trees having the goodnesses of fit that satisfy the preset conditions are extracted. Hence, it is possible to provide the RNA sequence analyzer, the RNA sequence analysis method, the program, and the recording medium capable of easily finding the RNA sequences potentially having the common structures.

INDUSTRIAL APPLICABILITY

As explained so far, the RNA sequence analyzer, the RNA sequence analysis method, the program, and the recording medium according to the present invention are suited, such as to the RNA secondary structure prediction, the RNA sequence analysis, and the gene analysis as well as developments of drugs using them.

**Claims**

1. An RNA sequence analyzer comprising:

   a grammar storage unit that stores structural topologies of RNA secondary structures with grammars corresponding to the structural topologies;
   a parsing unit that derives parse trees by applying an RNA sequence to the grammars;
   a goodness-of-fit calculation unit that calculates goodnesses of fit of the parse trees derived by the parsing unit;
   a sorting unit that sorts the parse trees having the goodnesses of fit that satisfy preset conditions in a descending order of the goodnesses of fit; and
   an output unit that outputs the parse trees sorted by the sorting unit as secondary structure candidates of the RNA sequence.

2. An RNA sequence analyzer comprising:

   a grammar storage unit that stores a structural topology of RNA secondary structures with a grammar corresponding to the structural topology;
   a parsing unit that derives parse trees by applying RNA sequences to the grammar;
   a goodness-of-fit calculation unit that calculates goodnesses of fit of the parse trees derived by the parsing unit; and
   an output unit that outputs the RNA sequences, from which the parse trees having the goodnesses of fit that satisfy preset conditions are derived, as RNA sequence candidates that could potentially form the secondary structures consistent with the structural topology.

**3.** An RNA sequence analyzer comprising:

a grammar storage unit that stores structural topologies of RNA secondary structures with grammars corresponding to the structural topologies;

a parsing unit that derives parse trees by applying RNA sequences to the grammars;

a goodness-of-fit calculation unit that calculates goodnesses of fit of the parse trees derived by the parsing unit;

an extraction unit that extracts the RNA sequences from which the parse trees having the goodnesses of fit that satisfy preset conditions are derived; and

a common structure matrix creation unit that displays the structural topologies and the RNA sequences in a two-dimensional matrix, that gives marks to lattice parts corresponding to the RNA sequences extracted by the extraction unit and the structural topologies in the two-dimensional matrix, and that thereby visualizes the structural topologies common to the RNA sequences.

**4.** An RNA sequence analyzer comprising:

a grammar storage unit that stores a structural topology of RNA secondary structures with a grammar corresponding to the structural topology;

an RNA sequence production unit that produces RNA sequences transcribed from a DNA sequence input by a user;

a parsing unit that derives parse trees by applying the grammar to the RNA sequences produced by the RNA sequence production unit;

a goodness-of-fit calculation unit that calculates goodnesses of fit of the parse trees derived by the parsing unit; and

a gene prediction unit that predicts parts of the DNA sequence corresponding to the RNA sequences, from which the parse trees having the goodnesses of fit that satisfy preset conditions are derived, as gene candidates.

**5.** An RNA sequence analyzer comprising:

a grammar storage unit that stores a structural topology of RNA secondary structures with a grammar corresponding to the structural topology;

a parsing unit that derives parse trees by applying the grammar to RNA sequences;

a goodness-of-fit calculation unit that calculates goodnesses of fit of the parse trees derived by the parsing unit; and

a similarity calculation unit that calculates a similarity among the RNA sequences based on the goodnesses of fit calculated by the goodness-of-fit calculation unit.

**6.** An RNA sequence analyzer comprising:

a grammar storage unit that stores structural topologies of RNA secondary structures with grammars corresponding to the structural topologies;

a parsing unit that derives parse trees by applying RNA sequences to the grammars;

a goodness-of-fit calculation unit that calculates goodnesses of fit of the parse trees derived by the parsing unit; and

an extraction unit that extracts the RNA sequences from which the parse trees having the goodnesses of fit that satisfy preset conditions are derived;

a goodness-of-fit matrix creation unit that creates a goodness-of-fit matrix which displays the structural topologies and the RNA sequences in a two-dimensional matrix, and which displays the goodnesses of fit on lattice parts corresponding to the RNA sequences extracted by the extraction unit and the structural topologies in. the two-dimensional matrix; and

a common structure extraction unit that sorts the structural topologies according to the goodnesses of fit for the goodness-of-fit matrix created by the goodness-of-fit matrix creation unit, that parses other RNA sequences based on the grammar corresponding to an order of the sorted structural topologies, and obtains the parse trees having optimum goodnesses of fit, and that extracts the other RNA sequences corresponding to the parse trees having the goodnesses of fit that satisfy the preset conditions.

**7.** An RNA sequence analysis method comprising:

a grammar storage step that stores structural topologies of RNA secondary structures with grammars corresponding to the structural topologies;

a parsing step that derives parse trees by applying an RNA sequence to the grammars;

a goodness-of-fit calculation step that calculates goodnesses of fit of the parse trees derived by the parsing step;

a sorting step that sorts the parse trees having the goodnesses of fit that satisfy preset conditions in a descending order of the goodnesses of fit; and

an output step that outputs the parse trees sorted by the sorting step as secondary structure candidates of the RNA sequence.

8. An RNA sequence analysis method comprising:

a grammar storage step that stores a structural topology of RNA secondary structures with a grammar corresponding to the structural topology;

a parsing step that derives parse trees by applying RNA sequences to the grammar;

a goodness-of-fit calculation step that calculates goodnesses of fit of the parse trees derived by the parsing step; and

an output step that outputs the RNA sequences, from which the parse trees having the goodnesses of fit that satisfy preset conditions are derived, as RNA sequence candidates that could potentially form the secondary structures consistent with the structural topology.

9. An RNA sequence analysis method comprising:

a grammar storage step that stores structural topologies of RNA secondary structures with grammars corresponding to the structural topologies;

a parsing step that derives parse trees by applying RNA sequences to the grammars;

a goodness-of-fit calculation step that calculates goodnesses of fit of the parse trees derived by the parsing step;

an extraction step that extracts the RNA sequences from which the parse trees having the goodness of fit that satisfy preset conditions are derived; and

a common structure matrix creation step that displays the structural topologies and the RNA sequences in a two-dimensional matrix, that gives marks to lattice parts corresponding to the RNA sequences extracted by the extraction step and the structural topologies in the two-dimensional matrix, and that thereby visualizes the structural topologies common to the RNA sequences.

10. An RNA sequence analysis method comprising:

a grammar storage step that stores a structural topology of RNA secondary structures with a grammar corresponding to the structural topology;

an RNA sequence production step that produces RNA sequences transcribed from a DNA sequence input by a user;

a parsing step that derives parse trees while applying the grammar to the RNA sequences produced by the RNA sequence production step;

a goodness-of-fit calculation step that calculates goodnesses of fit of the parse trees derived by the parsing step; and

a gene prediction step that predicts parts of the DNA sequence corresponding to the RNA sequences, from which the parse trees having the goodnesses of fit that satisfy preset conditions are derived, as gene candidates.

11. An RNA sequence analysis method comprising:

a grammar storage step that stores a structural topology of RNA secondary structures with a grammar corresponding to the structural topology;

a parsing step that derives parse trees by applying the grammar to RNA sequences;

a goodness-of-fit calculation step that calculates goodnesses of fit of the parse trees derived by the parsing step; and

a similarity calculation step that calculates a similarity among the RNA sequences based on the goodnesses of fit calculated by the goodness-of-fit calculation step.

**12.** An RNA sequence analysis method comprising:

a grammar storage step that stores structural topologies of RNA secondary structures with grammars corresponding to the structural topologies;

a parsing step that derives parse trees by applying RNA sequences to the grammars;

a goodness-of-fit calculation step that calculates goodnesses of fit of the parse trees derived by the parsing step; and

an extraction step that extracts the RNA sequences from which the parse trees having the goodnesses of fit that satisfy preset conditions are derived;

a goodness-of-fit matrix creation step that creates a goodness-of-fit matrix which displays the structural topologies and the RNA sequences in a two-dimensional matrix, and which displays the goodnesses of fit on lattice parts corresponding to the RNA sequences extracted by the extraction step and the structural topologies in the two-dimensional matrix; and

a common structure extraction step that sorts the structural topologies according to the goodnesses of fit for the goodness-of-fit matrix created by the goodness-of-fit matrix creation step, that parses other RNA sequences based on the grammar corresponding to an order of the sorted structural topologies, and obtains the parse trees having optimum goodnesses of fit, and that extracts the other RNA sequences corresponding to the parse trees having the goodnesses of fit that satisfy the preset conditions.

**13.** A computer program that makes a computer to execute an RNA sequence analysis method comprising:

a grammar storage step that stores structural topologies of RNA secondary structures with grammars corresponding to the structural topologies;

a parsing step that derives parse trees by applying an RNA sequence to the grammars;

a goodness-of-fit calculation step that calculates goodnesses of fit of the parse trees derived by the parsing step;

a sorting step that sorts the parse trees having the goodnesses of fit that satisfy preset conditions in a descending order of the goodnesses of fit; and

an output step that outputs the parse trees sorted by the sorting step as secondary structure candidates of the RNA sequence.

**14.** A computer program that makes a computer to execute an RNA sequence analysis method comprising:

a grammar storage step that stores a structural topology of RNA secondary structures with a grammar corresponding to the structural topology;

a parsing step that derives parse trees by applying RNA sequences to the grammar;

a goodness-of-fit calculation step that calculates goodnesses of fit of the parse trees derived by the parsing step; and

an output step that outputs the RNA sequences, from which the parse trees having the goodnesses of fit that satisfy preset conditions are derived, as RNA sequence candidates that could potentially form the secondary structures consistent with the structural topology.

**15.** A computer program that makes a computer to execute an RNA sequence analysis method comprising:

a grammar storage step that stores structural topologies of RNA secondary structures with grammars corresponding to the structural topologies;

a parsing step that derives parse trees by applying RNA sequences to the grammars;

a goodness-of-fit calculation step that calculates goodnesses of fit of the parse trees derived by the parsing step;

an extraction step that extracts the RNA sequences from which the parse trees having the goodnesses of fit that satisfy preset conditions are derived; and

a common structure matrix creation step that displays the structural topologies and the RNA sequences in a two-dimensional matrix, that gives marks to lattice parts corresponding to the RNA sequences extracted by the extraction step and the structural topologies in the two-dimensional matrix, and that thereby visualizes the structural topologies common to the RNA sequences.

**16.** A computer program that makes a computer to execute an RNA sequence analysis method comprising:

a grammar storage step that stores a structural topology of RNA secondary structures with a grammars corresponding to the structural topology;

an RNA sequence production step that produces RNA sequences transcribed from a DNA sequence input by a user;

a parsing step that derives parse trees by applying the grammar to the RNA sequences produced by the RNA sequence production step;

a goodness-of-fit calculation step that calculates goodnesses of fit of the parse trees derived by the parsing step; and

a gene prediction step that predicts parts of the DNA sequence corresponding to the RNA sequences, from which the parse trees having the goodnesses of fit that satisfy preset conditions are derived, as gene candidates.

17. A computer program that makes a computer to execute an RNA sequence analysis method comprising:

a grammar storage step that stores a structural topology of RNA secondary structures with a grammar corresponding to the structural topology;

a parsing step that derives parse trees by applying the grammar to RNA sequences;

a goodness-of-fit calculation step that calculates goodnesses of fit of the parse trees derived by the parsing step; and

a similarity calculation step that calculates a similarity among the RNA sequences based on the goodnesses of fit calculated by the goodness-of-fit calculation step.

18. A computer program that makes a computer to execute an RNA sequence analysis method comprising:

a grammar storage step that stores structural topologies of RNA secondary structures with grammars corresponding to the structural topologies;

a parsing step that derives parse trees by applying RNA sequences to the grammars;

a goodness-of-fit calculation step that calculates goodnesses of fit of the parse trees derived by the parsing step; and

an extraction step that extracts the RNA sequences from which the parse trees having the goodness of fit that satisfy preset conditions are derived;

a goodness-of-fit matrix creation step that creates a goodness-of-fit matrix which displays the structural topologies and the RNA sequences in a two-dimensional matrix, and which displays the goodnesses of fit on lattice parts corresponding to the RNA sequences extracted by the extraction step and the structural topologies in the two-dimensional matrix; and

a common structure extraction step that sorts the structural topologies according to the goodnesses of fit for the goodness-of-fit matrix created by the goodness-of-fit matrix creation step, that parses other RNA sequences based on the grammar corresponding to an order of the sorted structural topologies, and obtains the parse trees having optimum goodnesses of fit, and that extracts the other RNA sequences corresponding to the parse trees having the goodnesses of fit that satisfy the preset conditions.

19. A computer readable recording medium storing a computer program that makes a computer to execute an RNA sequence analysis method comprising:

a grammar storage step that stores a structural topologies of RNA secondary structures with grammars corresponding to the structural topologies;

a parsing step that derives parse trees by applying an RNA sequence to the grammars;

a goodness-of-fit calculation step that calculates goodnesses of fit of the parse trees derived by the parsing step;

a sorting step that sorts the parse trees having the goodnesses of fit that satisfy preset conditions in a descending order of the goodnesses of fit; and

an output step that outputs the parse trees sorted by the sorting step as secondary structure candidates of the RNA sequence.

20. A computer readable recording medium storing a computer program that makes a computer to execute an RNA sequence analysis method comprising:

a grammar storage step that stores a structural topology of RNA secondary structures with a grammar corresponding to the structural topology;

a parsing step that derives parse trees by applying RNA sequences to the grammar;

a goodness-of-fit calculation step that calculates goodnesses of fit of the parse trees derived by the parsing step; and

an output step that outputs the RNA sequences, from which the parse trees having the goodnesses of fit that satisfy preset conditions are derived, as RNA sequence candidates that could potentially form the secondary structures consistent with the structural topology.

21. A computer readable recording medium storing a computer program that makes a computer to execute an RNA sequence analysis method comprising:

a grammar storage step that stores structural topologies of RNA secondary structures with grammars corresponding to the structural topologies;

a parsing step that derives parse trees by applying RNA sequences to the grammars;

a goodness-of-fit calculation step that calculates goodnesses of fit of the parse trees derived by the parsing step;

an extraction step that extracts the RNA sequences from which the parse trees having the goodness of fit that satisfy preset conditions are derived; and

a common structure matrix creation step that displays the structural topologies and the RNA sequences in a two-dimensional matrix, that gives marks to lattice parts corresponding to the RNA sequences extracted by the extraction step and the structural topologies in the two-dimensional matrix, and that thereby visualizes the structural topologies common to the RNA sequences.

22. A computer readable recording medium storing a computer program that makes a computer to execute an RNA sequence analysis method comprising:

a grammar storage step that stores a structural topology of RNA secondary structures with a grammar corresponding to the structural topology;

an RNA sequence production step that produces RNA sequences transcribed from a DNA sequence input by a user;

a parsing step that derives parse trees by applying the grammar to the RNA sequences produced by the RNA sequence production step;

a goodness-of-fit calculation step that calculates goodnesses of fit of the parse trees derived by the parsing step; and

a gene prediction step that predicts parts of the DNA sequence corresponding to the RNA sequences, from which the parse trees having the goodnesses of fit that satisfy preset conditions are derived, as gene candidates.

23. A computer readable recording medium storing a computer program that makes a computer to execute an RNA sequence analysis method comprising:

a grammar storage step that stores a structural topology of RNA secondary structures with a grammar corresponding to the structural topology;

a parsing step that derives parse trees by applying the grammar to RNA sequences;

a goodness-of-fit calculation step that calculates goodnesses of fit of the parse trees derived by the parsing step; and

a similarity calculation step that calculates a similarity among the RNA sequences based on the goodnesses of fit calculated by the goodness-of-fit calculation step.

24. A computer readable recording medium storing a computer program that makes a computer to execute an RNA sequence analysis method comprising:

a grammar storage step that stores structural topologies of RNA secondary structures with grammars corresponding to the structural topologies;

a parsing step that derives parse trees by applying RNA sequences to the grammars;

a goodness-of-fit calculation step that calculates goodnesses of fit of the parse trees derived by the parsing step; and

an extraction step that extracts the RNA sequences from which the parse trees having the goodnesses of fit that satisfy preset conditions;

a goodness-of-fit matrix creation step that creates a goodness-of-fit matrix which displays the structural topologies and the RNA sequences in a two-dimensional matrix, and which displays the goodnesses of fit on lattice parts corresponding to the RNA sequences extracted by the extraction step and the structural topologies in the two-dimensional matrix; and

a common structure extraction step that sorts the structural topologies according to the goodnesses of fit for the goodness-of-fit matrix created by the goodness-of-fit matrix creation step, that parses other RNA sequences based on the grammar corresponding to an order of the sorted structural topologies, and obtains the parse trees having optimum goodnesses of fit, and that extracts the other RNA sequences corresponding to the parse trees having the goodnesses of fit that satisfy the preset conditions.

# FIG.1

(a)　　　　　(b)　　　　　(c)　　　　　(d)

HAIRPIN LOOP　　　BULGE LOOP　　　INTERNAL LOOP　　　PSEUDOKNOTS

# FIG.2

### (a) RNA SEQUENCE

| C | A | G | G | A | A | A | C | U | G | G | G | U | G | C | A | A | A | C | C |
|---|---|---|---|---|---|---|---|---|---|---|---|---|---|---|---|---|---|---|---|

### (b) SECONDARY STRUCTURE (STEM STRUCTURE)

```
      5'            3'
        C—G    G—C
        A—U    G—C
        G—C    U—A
        G   A  G   A
         A A    C A
```

### (c) PARSE TREE

```
C A G G A A A C U G G G U G C A A A C C
```

# FIG.3

| STRUCTURAL TOPOLOGY | CORRESPOND | GRAMMAR (T, N, P) |

PSEUDOKNOTS $\longleftrightarrow (\{A,T,G,C\},N_1,P_1)$

HAIRPIN LOOP $\longleftrightarrow (\{A,T,G,C\},N_2,P_2)$

$\vdots \qquad\qquad\qquad\qquad \vdots$

# FIG.4

TREE GRAMMAR
PARSER

RNA SEQUENCE

PARSING

DERIVE

$e_1$ $e_2$ $e_3$ $\cdots$

ENERGY
CALCULATION

ZERO OR MORE
PARSE TREE

(T,N,P)
GRAMMAR

# FIG.5

EP 1 471 444 A1

# FIG.6

GRAMMAR DATABASE 106b

| STRUCTURAL TOPOLOGY | GRAMMAR | | |
|---|---|---|---|
| | TERMINAL SYMBOL T | NONTERMINAL SYMBOL N | PRODUCTION RULES P |
| PSEUDOKNOTS | | $N_1$ | $P_1$ |
| HAIRPIN LOOP | | $N_2$ | $P_2$ |
| INTERNAL LOOP | {A,T,G,C} | $N_3$ | $P_3$ |
| MULTI-BRANCHED LOOP | | $N_4$ | $P_4$ |
| BULGE LOOP | | $N_5$ | $P_5$ |
| ⋮ | | ⋮ | ⋮ |

EP 1 471 444 A1

# FIG.7

STRUCTURE
PREDICTION SECTION          102a

PARSING

RNA SEQUENCE
HAVING UNKNOWN
STRUCTURE

SA-1

GOODNESS-
OF-FIT
CALCULATION

DERIVE

SA-4

$e_1$  $e_2$  $e_3$  . . .

PARSE TREE

FIT    SA-3

106b
FETCH

GRAMMAR  . . .

GRAMMAR
DB

SA-2

(T,N,P)

EP 1 471 444 A1

# FIG.8

106a

RNA
SEQUENCE
DB

SEARCH RESULT

GRAMMAR 1
(HAIRPIN LOOP)

▯▯▯▯▯▯▯▯▯▯▯▯▯

STRUCTURE
PREDICTION SECDION      102a

FETCH       SB-1

▯▯▯▯▯▯▯▯▯▯▯▯▯

RNA SEQUENCES

SB-2

PARSING

GOODNESS-
OF-FIT
CALCULATION

DERIVE

SB-4

SB-5

PARSE TREE

FIT    SB-3

GRAMMAR

(T,N,P)

40

# FIG.9

OUTPUT COMMON
STRUCTURE MATRIX

GRAMMAR
(STRUCTURAL TOPOLOGY)

| SEQUENCE | 1 | 2 | 3 | 4 | 5 | 6 | 7 |
|---|---|---|---|---|---|---|---|
| 1 | | ■ | | | | | |
| 2 | | ■ | | ■ | | | ■ |
| 3 | | ■ | | | | | |
| 4 | | ■ | | | ■ | | |
| 5 | | ■ | | | | | |

106a

RNA
SEQUENCE
DB

STRUCTURE
PREDICTION
SECTION

102a

FETCH

SC-1

SC-2

RNA SEQUENCE

PARSING

GOODNECS-
OF-FIT
CALCULATION

DERIVE

SC-5

PARSE TREE

SC-6

SC-4

106b

FETCH

GRAMMAR

GRAMMAR
DB

SC-3

(T,N,P)

. . .

# FIG.10

RNA SEQUENCE

1 ▯▯▯▯▯▯▯▯▯▯▯▯▯

2 ▯▯▯▯▯▯▯▯▯▯▯▯▯

⬇ SE-1

STRUCTURE PREDICTION SECTION 102a

| PARSING |
| GOODNESS-OF-FIT CALCULATION |

SE-4 ⬇

GOODNESS OF FIT FOR EACH STRUCTURAL TOPOLOGY

$E_1$ | $e_1$ | $e_2$ | $e_3$ | $e_4$ | $e_5$ | $e_6$ | $e_7$ | ...

$E_2$ | $e_1$ | $e_2$ | $e_3$ | $e_4$ | $e_5$ | $e_6$ | $e_7$ | ...

SE-5 →

SIMILARITY CALCULATION SECTION 102d

| SIMILARITY CALCULATION |

⬇ SE-6

( SIMILARITY )

SE-3 ↑

106b

GRAMMAR DB

SE-2 →

GRAMMAR ...

(T,N,P)

EP 1 471 444 A1

# FIG.11

DNA SEQUENCE

AUTOMATIC
TRANSFORMATION (SF-1)

PREDICTED
RNA SEQUENCE

SF-2

STRUCTURE
PREDICTION SECTION
102a

PARSING

GOODNESS-
OF-FIT
CALCULATION

DERIVE
SF-5

PARSE TREE

SF-6

HIGH PROBABILITY
OF BEING RNA

SF-4

GRAMMAR

106b

GRAMMAR
DB       SF-3

(T,N,P)

...

# FIG.12

$R_1$

$R_2$

$S_1$

$S_2$

P=2

NOT COUNTED AS P AND
CANNOT BELONG TO $S_1$ AND $S_2$

FOR j =1, 2 AND k = 1, 2, 4, 6, 8, 9,
$S_j$ IS A VECTOR THAT IS A COLLECTION OF $R_j[k]$

# FIG.13

(EXAMPLES OF RNA SECONDARY STRUCTURE TOPOLOGY)

$L(a)$        $L_1(b)$      $L_2(b)$

$H(a)$     $H_1(b)$    $H_2(b)$

$I(b)$

(a) STEM LOOP     (b) DOUBLE-PARALLEL
-STEM LOOP

# FIG. 14

(PARSE TREES AND SECONDARY STRUCTURES OF S₁)

(1)

(2)

# FIG.15

(FREE ENERGIES OF BASE PAIRS [kcal/mol])

| 3'-SIDE BASE PAIR | 5'-SIDE BASE PAIR | | | | |
|---|---|---|---|---|---|
| | gu | au | ua | cg | gc |
| gu | -0.5 | -0.5 | -0.7 | -1.5 | -1.3 |
| au | -0.5 | -0.9 | -1.1 | -1.8 | -2.3 |
| ua | -0.7 | -0.9 | -0.9 | -1.7 | -2.1 |
| cg | -1.9 | -2.1 | -2.3 | -2.9 | -3.4 |
| gc | -1.5 | -1.7 | -1.8 | -2.0 | -2.9 |

# FIG.16

(FREE ENERGIES OF LOOPS [kcal/mol])

| LOOP TYPE | LOOP SIZE | | | | | | | | | | | | |
|---|---|---|---|---|---|---|---|---|---|---|---|---|---|
| | 1 | 2 | 3 | 4 | 5 | 6 | 7 | 8 | 9 | 10 | 12 | 14 | 16 |
| BULGE LOOP | 3.3 | 5.2 | 6.0 | 6.7 | 7.4 | 8.2 | 9.1 | 10.0 | 10.5 | 11.0 | 11.8 | 12.5 | 13.0 |
| HAIRPIN LOOP | - | - | 7.4 | 5.9 | 4.4 | 4.3 | 4.1 | 4.1 | 4.2 | 4.3 | 4.9 | 5.6 | 6.1 |
| INTERNAL LOOP | - | 0.8 | 1.3 | 1.7 | 2.1 | 2.5 | 2.6 | 2.8 | 3.1 | 3.6 | 4.4 | 5.1 | 5.6 |

# FIG.17

(OPTIMUM PARSE TREES AND SECONDARY STRUCTURES OF $S_2$)

(1) DERIVATION BASED ON $G_1$

(2) DERIVATION BASED ON $G_2$

# FIG.18

| RANKING | TOPOLOGY | GOODNESS OF FIT | SECONDARY STRUCTURE |
|---------|----------|-----------------|---------------------|
| 1 | STEM LOOP $T_1$ | 13.7 | SECONDARY STRUCTURE ILLUSTRATED IN Fig. 18(1) |
| 2 | DOUBLE-PARALLEL-STEM LOOP $T_2$ | 6.7 | SECONDARY STRUCTURE ILLUSTRATED IN Fig. 18(2) |

# FIG.19

| SEQUENCE | GOODNESS OF FIT | SECONDARY STRUCTURE |
|---|---|---|
| $s_2$ | 6.7 | SECONDARY STRUCTURE ILLUSTRATED IN Fig. 18(2) |

## FIG.20

|      | $T_1$ | $T_2$ |
|------|-------|-------|
| $s_1$ | 1.4  | -     |
| $s_2$ | 13.6 | 6.7   |

# FIG.21

### (OPTIMUM SECONDARY STRUCTURE OF s)

(1)

(2)

# FIG.22

|   | $T_1$ | $T_2$ |
|---|-------|-------|
| s | -     | 5.1   |

# FIG.23

| SEQUENCE | GOODNESS OF FIT | TOPOLOGY (SCORE) |
|:---:|:---:|:---:|
| $s_2$ | 6.7 | $T_2(5.1)$ |

# INTERNATIONAL SEARCH REPORT

| International application No. |
|---|
| PCT/JP03/00011 |

| A. CLASSIFICATION OF SUBJECT MATTER |
|---|
| Int.Cl⁷ G06F17/30, C12Q1/68, C12N15/00, G01N33/50 |

According to International Patent Classification (IPC) or to both national classification and IPC

| B. FIELDS SEARCHED |
|---|

Minimum documentation searched (classification system followed by classification symbols)
Int.Cl⁷ G06F17/30, C12Q1/68, C12N15/00, G01N33/50

Documentation searched other than minimum documentation to the extent that such documents are included in the fields searched

| Jitsuyo Shinan Koho | 1922–1996 | Toroku Jitsuyo Shinan Koho | 1994–2003 |
|---|---|---|---|
| Kokai Jitsuyo Shinan Koho | 1971–2003 | Jitsuyo Shinan Toroku Koho | 1996–2003 |

Electronic data base consulted during the international search (name of data base and, where practicable, search terms used)
JICST FILE(JOIS), WPI, INSPEC(DIALOG)

| C. DOCUMENTS CONSIDERED TO BE RELEVANT |
|---|

| Category* | Citation of document, with indication, where appropriate, of the relevant passages | Relevant to claim No. |
|---|---|---|
| X | YOSHIZAWA et al., "RNA Niji Kozo Yosoku Shien System no Kaihatsu to Jisso", The Society for Artificial Intelligence Jinko Chino Kisoron Kenkyukai(Dai 32 Kai) Shiryo, 26 March, 1998 (26.03. 98), pages 29 to 35, particularly, page 31 | 1,2,4,5,7, 8,10,11,13, 14,16,17,19, 20,22,23 |
| A | | 3,6,9,12,15, 18,21,24 |
| A | UEMURA, Y. et al., Grammatically Modeling and Predicting RNA Secondary Structures. Genome Informatics Series, Proceedings Genome Informatics Workshop, 11 December, 1995 (11.12.95), No.6, pages 67 to 76 | 1-24 |

☐ Further documents are listed in the continuation of Box C.　　☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 17 February, 2003 (17.02.03) | 04 March, 2003 (04.03.03) |

| Name and mailing address of the ISA/ | Authorized officer |
|---|---|
| Japanese Patent Office | |
| Facsimile No. | Telephone No. |

Form PCT/ISA/210 (second sheet) (July 1998)